# EUROPEAN PATENT APPLICATION

(11) **EP 4 660 205 A1**
(43) Date of publication of application: **10.12.2025**
(21) Application number: 24749557.5
(22) Date of filing: 22.01.2024
(51) Int. Cl.: C07K 16/46, C07K 16/28, A61K 39/395, A61P 1/00, A61P 29/00, A61P 13/12, A61P 11/00, A61P 19/02, A61P 17/06

(54) **MULTIPLE ANTIBODIES AGAINST HUMAN IL-36R AND/OR HUMAN IL-1R3, AND USES THEREOF**

(30) Priority: 30.01.2023 CN 202310103046
(71) Applicant: Beijing Wisdomab Biotechnology Co., Ltd, Beijing 101111 (CN); Genrix(Shanghai) Biopharmaceutical Co., Ltd., Shanghai 201203 (CN); Chongqing Genrix Biopharmaceutial Co., Ltd., Chongqing 401319 (CN)
(72) Inventor: HAO, Xiaobo, Beijing 100163 (CN); LIU, Zhigang, Beijing 100039 (CN); ZHOU, Xiaowei, Beijing 100039 (CN); FU, Xinlei, Beijing 101103 (CN); LIU, Yulan, Beijing 100176 (CN); HU, Junjie, Beijing 100176 (CN)
(74) Representative: Lavoix
(86) International application number: PCT/CN2024/073397
(87) International publication number: WO 2024/160076

(57) **Abstract**

Provided in the present application are a bispecific antibody against human IL-36R and human IL-1R3, a monoclonal antibody binding to human IL-36R, a monoclonal antibody binding to human IL-1R3, and the uses thereof.

## Description

### CROSS REFERECE TO RELATED APPLICATION

The present application claims priority to Chinese Patent Application No. 202310103046.5, filed on January 30, 2023, which is incorporated herein by reference in its entirety.

### TECHNICAL FIELD

The present invention relates generally to the field of genetic engineering and antibody medicine; In particular, the present application relates to a bispecific antibody against human IL-36R and human IL-1R3, a monoclonal antibody binding to human IL-36R, a monoclonal antibody binding to human IL-1R3, and uses thereof.

### BACKGROUND

Interleukin 36 (IL-36) is mainly expressed in epithelial cells and immune cells and includes three pro-inflammatory cytokines IL-36α (IL-1F6), IL-36β (IL-1F8) and IL-36γ (IL1F9) and two anti-inflammatory cytokines IL-36Ra (IL-1F5) and IL-38 (IL-1F10)¹⁻². The pro-inflammatory IL-36 factors (IL-36α, IL-36β, and IL-36γ) bind first to a first receptor IL-36R (IL-1Rrp2) on the cell surface, and then recruit a second receptor IL-1R3 (IL-1RAcP) to form a ternary complex, which activates downstream NF-κB and MAPK signals via the intracellular TIR domain ³. The anti-inflammatory IL-36 factors (IL-36Ra and IL-38) bind to IL-36R and thus block the binding of the pro-inflammatory IL-36 factors, thereby blocking downstream signaling and maintaining balance of inflammatory responses in the body.

Overexpression of IL-36α, IL-36β and IL-36γ or under-expression of IL-36Ra and IL-18 may lead to dysregulation of the IL-36 signaling pathway, resulting in various autoimmune and inflammatory diseases, such as psoriasis vulgaris, inflammatory bowel disease, renal injury and inflammation, pneumonia, arthritis, hidradenitis suppurative, generalized pustular psoriasis, palmoplantar pustular psoriasis, and the like ²⁻⁴. Blocking the IL-36 signaling pathway has shown significant therapeutic effects in clinical studies for the treatment of pustular psoriasis ⁵⁻⁶, and has also shown positive therapeutic effects on patients with severe palmoplantar pustular psoriasis ⁷.

Pustular psoriasis is a special type of psoriasis, with clinical signs of aseptic pustules in addition to erythema, and has three phenotypes: generalized pustular psoriasis, palmoplantar pustular psoriasis, and acrodermatitis continua ⁸. Current therapies for treating pustular psoriasis include oral retinoids and topical steroids, with limited efficacy and with side effects.

Bispecific antibody (BsAb), as a kind of artificial antibodies, contains two different antigen-binding sites and is capable of bridging target cells with functional molecules (cells) and stimulating a directed immune response. It has become a research hotspot in the field of antibody engineering, and has a broad application prospect in immunotherapy of diseases.

There is a need in the art to develop and apply novel antibodies against human IL-36R and/or human IL-1R3. Therefore, given that the antibodies against human IL-36R and/or human IL-1R3 have wide applicability, and due to clinical needs, it is of significant biological and medical importance to explore and develop the novel antiboties against human IL-36R and/or human IL-1R3.

### SUMMARY OF THE INVENTION

In a first aspect, the present application provides a bispecific antibody comprising a first antigen-binding fragment that binds to human IL-36R and a second antigen-binding fragment that binds to human IL-1R3.

In some embodiments of the first aspect, the bispecific antibody is capable of inhibiting an IL-36-mediated inflammatory response.

In some embodiments of the first aspect, the first antigen-binding fragment comprises:
HCDR1 having the amino acid sequence SSWIH (SEQ ID NO: 1), HCDR2 having the amino acid sequence EINPGNVRSNYNENFRN (SEQ ID NO: 2), HCDR3 having the amino acid sequence INYGEPYFAS (SEQ ID NO: 3), LCDR1 having the amino acid sequence RASQSVIGSYLA (SEQ ID NO: 4), LCDR2 having the amino acid sequence SASKLAS (SEQ ID NO: 5), and LCDR3 having the amino acid sequence QQDTRYPIT (SEQ ID NO: 6); or
HCDR1 having the amino acid sequence SSWIH (SEQ ID NO: 1), HCDR2 having the amino acid sequence EINPGSGRSNYNENFQG (SEQ ID NO: 7), HCDR3 having the amino acid sequence VFYGEPYFPF (SEQ ID NO: 8), LCDR1 having the amino acid sequence RASQSVIYSYLA (SEQ ID NO: 9), LCDR2 having the amino acid sequence SASKRAS (SEQ ID NO: 10), and LCDR3 having the amino acid sequence QQHAGIPVT (SEQ ID NO: 11);
wherein the amino acid sequences of the HCDRs and the LCDRs are defined according to Kabat.

In some embodiments of the first aspect, the second antigen-binding fragment comprises:
HCDR1 having the amino acid sequence SYAMS (SEQ ID NO: 12), HCDR2 having the amino acid sequence EISRGGSHTYYPDTVTG (SEQ ID NO: 13), HCDR3 having the amino acid sequence SYGNYPFDY (SEQ ID NO: 14), LCDR1 having the amino acid sequence RASQSVIGSYLA (SEQ ID NO: 4), LCDR2 having the amino acid sequence SASKLAS (SEQ ID NO: 5), and LCDR3 having the amino acid sequence QQDTRYPIT (SEQ ID NO: 6); or
HCDR1 having the amino acid sequence SYAMS (SEQ ID NO: 12), HCDR2 having the amino acid sequence EISRGGSHTYYPDTVTG (SEQ ID NO: 13), HCDR3 having the amino acid sequence SYGNYPFDY (SEQ ID NO: 14), LCDR1 having the amino acid sequence SASSSVSYMY (SEQ ID NO: 15), LCDR2 having the amino acid sequence DTSNLAS (SEQ ID NO: 16), and LCDR3 having the amino acid sequence QQWSSYPLT (SEQ ID NO: 17);
wherein the amino acid sequences of the HCDRs and the LCDRs are defined according to Kabat.

In some embodiments of the first aspect, the first antigen-binding fragment comprises a heavy chain variable region having the amino acid sequence set forth in SEQ ID NO: 18, and a light chain variable region having the amino acid sequence set forth in SEQ ID NO: 19; or
the first antigen-binding fragment comprises a heavy chain variable region having the amino acid sequence set forth in SEQ ID NO: 20, and a light chain variable region having the amino acid sequence set forth in SEQ ID NO: 21.

In some embodiments of the first aspect, the second antigen-binding fragment comprises a heavy chain variable region having the amino acid sequence set forth in SEQ ID NO: 22, and a light chain variable region having the amino acid sequence set forth in SEQ ID NO:19; or
the second antigen-binding fragment comprises a heavy chain variable region having the amino acid sequence set forth in SEQ ID NO: 22, and a light chain variable region having the amino acid sequence set forth in SEQ ID NO:23.

In some embodiments of the first aspect, the first antigen-binding fragment comprises a heavy chain variable region set forth in SEQ ID NO: 18 and a light chain variable region set forth in SEQ ID NO: 19, and the second antigen-binding fragment comprises a heavy chain variable region set forth in SEQ ID NO: 22 and a light chain variable region set forth in SEQ ID NO: 19; or
the first antigen-binding fragment comprises a heavy chain variable region set forth in SEQ ID NO: 20 and a light chain variable region set forth in SEQ ID NO: 21, and the second antigen-binding fragment comprises a heavy chain variable region set forth in SEQ ID NO: 22 and a light chain variable region set forth in SEQ ID NO: 23.

In some embodiments of the first aspect, the bispecific antibody is an IgG1 antibody comprising a first heavy chain constant region and a second heavy chain constant region, wherein amino acids at positions 354 and 366 of the first heavy chain constant region are C and W, respectively, and amino acids at positions 349, 366, 368, and 407 of the second heavy chain constant region are C, S, A, and V, respectively; the amino acid positions of the antibody constant regions are determined according to EU numbering.

In some embodiments of the first aspect, the bispecific antibody is an IgG1 antibody comprising a first heavy chain constant region and a second heavy chain constant region, wherein amino acids at positions 234, 235, and 331 of the first heavy chain constant region and the second heavy chain constant region are F, E, and S, respectively; the amino acid positions of the antibody constant regions are determined according to EU numbering.

In some embodiments of the first aspect, the first antigen-binding fragment and the second antigen-binding fragment are independently selected from a single chain fragment variable (scFv) or a Fab fragment.

In a second aspect, the present application provides a monoclonal antibody that binds to human IL-36R, comprising
HCDR1 set forth in SEQ ID NO: 1,
HCDR2 set forth in SEQ ID NO: 7,
HCDR3 set forth in SEQ ID NO: 8,
LCDR1 set forth in SEQ ID NO: 9,
LCDR2 set forth in SEQ ID NO: 10, and
LCDR3 set forth in SEQ ID NO: 11; or
HCDR1 set forth in SEQ ID NO: 1,
HCDR2 set forth in SEQ ID NO: 2,
HCDR3 set forth in SEQ ID NO: 3,
LCDR1 set forth in SEQ ID NO: 4,
LCDR2 set forth in SEQ ID NO: 5, and
LCDR3 set forth in SEQ ID NO: 6; or
HCDR1 set forth in SEQ ID NO: 1,
HCDR2 set forth in SEQ ID NO: 7,
HCDR3 set forth in SEQ ID NO: 8,
LCDR1 set forth in SEQ ID NO: 4,
LCDR2 set forth in SEQ ID NO: 5, and
LCDR3 set forth in SEQ ID NO: 6;
wherein the amino acid sequences of the HCDRs and the LCDRs are defined according to Kabat.

In a third aspect, the present application provides a monoclonal antibody that binds to human IL-1R3, comprising
HCDR1 set forth in SEQ ID NO: 12,
HCDR2 set forth in SEQ ID NO: 13,
HCDR3 set forth in SEQ ID NO: 14,
LCDR1 set forth in SEQ ID NO: 15,
LCDR2 set forth in SEQ ID NO: 16, and
LCDR3 set forth in SEQ ID NO: 17;
wherein the amino acid sequences of the HCDRs and the LCDRs are defined according to Kabat.

In a fourth aspect, the present application provides a pharmaceutical composition comprising the bispecific antibody of the first aspect, or the monoclonal antibody of the second or third aspect, and a pharmaceutically acceptable excipient, diluent, or carrier.

In a fifth aspect, the present application provides use of the bispecific antibody of the first aspect, the monoclonal antibody of the second or third aspect, or the pharmaceutical composition of the fourth aspect in the manufacture of a medicament for the prevention or treatment of an IL-36-mediated disease.

### BRIEF DESCRIPTION OF DRAWINGS

Figure 1 shows the ability of an anti-IL-36R antibody to inhibit IL-36 from stimulating SEAP secretion by HEK-Blue^{™} IL-36 cells. Panel A shows the inhibition of IL-36α, panel B shows the inhibition of IL-36β, and panel C shows the inhibition of IL-36γ.
Figure 2 shows the ability of the IL-36R antibody to inhibit IL-36 from stimulating IL-8 secretion by HaCAT cells. Panel A shows the inhibition of IL-36α, panel B shows the inhibition of IL-36β, and panel C shows the inhibition of IL-36γ.
Figure 3 shows the ability of a mouse IL-1R3 monoclonal antibody to recognize IL-1R3 on the surface of HaCAT cells.
Figure 4 shows the ability of a humanized IL-1R3 antibody to recognize IL-1R3 on the surface of HaCAT cells.
Figure 5 shows the ability of an IL-36R×IL-1R3 Crossmab bispecific antibody to inhibit IL-36 from stimulating IL-8 secretion by HaCAT cells. Panel A shows the inhibition of IL-36α, panel B shows the inhibition of IL-36β, and panel C shows the inhibition of IL-36γ.
Figure 6 shows the ability of a common light chain bispecific antibody to inhibit IL-36 from stimulating IL-8 secretion by HaCAT cells. Panel A shows the inhibition of IL-36α, panel B shows the inhibition of IL-36β, and panel C shows the inhibition of IL-36γ.
Figure 7 shows the ability of the IL-36R monoclonal antibody and the IL-36R×IL-1R3 common light chain bispecific antibody to inhibit IL-36γ from stimulating IL-8 secretion by human monocytes.
Figure 8 shows the ability of the IL-36R×IL-1R3 common light chain bispecific antibody to inhibit IL-33 and IL-12 from stimulating IL-8 secretion by human NK cells.
Figure 9 shows the results of the anti-IL-36R×IL-1R3 common light chain bispecific antibody for inhibiting IL-1 from stimulating IL-8 secretion by MRC-5 cells. Panel A shows the inhibition of IL-1α, and panel B shows the inhibition of IL-1β.

### DESCRIPTION OF SEQUENCES

SEQ ID NO: 1 shows the amino acid sequence of CDR1 of the heavy chain variable regions R49E11VH-h3 and R72B7.
SEQ ID NOs: 2-3 show the amino acid sequences of CDR2 and CDR3 of the heavy chain variable region R72B7, respectively.
SEQ ID NOs: 4-6 show the amino acid sequences of CDR1, CDR2 and CDR3 of the light chain variable region L36E9VK, respectively.
SEQ ID NOs: 7-8 show the amino acid sequences of CDR2 and CDR3 of the heavy chain variable region R49E11 VH-h3, respectively.
SEQ ID NOs: 9-11 show the amino acid sequences of CDR1, CDR2 and CDR3 of the light chain variable region L26B11VK, respectively.
SEQ ID NOs: 12-14 show the amino acid sequences of CDR1, CDR2 and CDR3 of the heavy chain variable region R1B9VH-h1, respectively.
SEQ ID NOs: 15-17 show the amino acid sequences of CDR1, CDR2 and CDR3 of the light chain variable region R1B9VK-h1, respectively.
SEQ ID NO: 18 shows the amino acid sequence of the heavy chain variable region R72B7.
SEQ ID NO: 19 shows the amino acid sequence of the light chain variable region L36E9VK.
SEQ ID NO: 20 shows the amino acid sequence of the heavy chain variable region R49E11VH-h3.
SEQ ID NO: 21 shows the amino acid sequence of the light chain variable region L26B11 VK.
SEQ ID NO: 22 shows the amino acid sequence of the heavy chain variable region RIB9VH-h1.
SEQ ID NO: 23 shows the amino acid sequence of the light chain variable region R1B9VK-h1.
SEQ ID NO: 24 shows the amino acid sequence of the extracellular domain of human *(homo sapiens)* IL-36R (hIL-36R).
SEQ ID NO: 25 shows the amino acid sequence of the extracellular domain of monkey (*Macaca fascicularis*) IL-36R (mfIL-36R).
SEQ ID NO: 26 shows the amino acid sequence of the extracellular domain of human *(homo sapiens)* IL-1R3 (hIL-1R3).
SEQ ID NO: 27 shows the amino acid sequence of the extracellular domain of monkey (*Macaca fascicularis*) IL-1R3 (mflL-1R3).
SEQ ID NO: 28 shows the amino acid sequence of the mature peptide of human *(homo sapiens)* IL-1α (hIL-1α).
SEQ ID NO: 29 shows the amino acid sequence of the mature peptide of human *(homo sapiens)* IL-1β (hIL-1β).
SEQ ID NO: 30 shows the amino acid sequence of the mature peptide of human *(homo sapiens)* IL-33 (hIL-33).
SEQ ID NO: 31 shows the amino acid sequence of the mature peptide of human *(homo sapiens)* IL-36α (hIL-36α).
SEQ ID NO: 32 shows the amino acid sequence of the mature peptide of human *(homo sapiens)* IL-36β (hIL-36β).
SEQ ID NO: 33 shows the amino acid sequence of the mature peptide of human *(homo sapiens)* IL-36γ (hIL-36γ).
SEQ ID NO: 34 shows the amino acid sequence of the His tag (His).
SEQ ID NO: 35 shows the amino acid sequence of the Fc fragment (mFc) of mouse (*mus musculus*) IgG2a antibody.
SEQ ID NO: 36 shows the amino acid sequence of the heavy chain constant region of human (*homo sapiens*) IgG1 subtype.
SEQ ID NO: 37 shows the amino acid sequence of human IgG1 subtype heavy chain constant region mutant IgG1m3.
SEQ ID NO: 38 shows the amino acid sequence of the human IgG1 subtype heavy chain constant region mutant IgG1m3-H1.
SEQ ID NO: 39 shows the amino acid sequence of human IgG1 subtype heavy chain constant region mutant IgG1m3-K.
SEQ ID NO: 40 shows the amino acid sequence of the light chain constant region of human (*homo sapiens*) kappa subtype.
SEQ ID NO: 41 shows the amino acid sequence of the light chain constant region of human (*homo sapiens*) lamda subtype.
SEQ ID NO: 42 shows the amino acid sequence of the heavy chain variable region of monoclonal antibody Spesolimab.
SEQ ID NO: 43 shows the amino acid sequence of the light chain variable region of monoclonal antibody Spesolimab.
SEQ ID NO: 44 shows the amino acid sequence of R49E11VH-h3-IgG1m3-H1.
SEQ ID NO: 45 shows the amino acid sequence of R1B9VH-h1-IgG1m3-K.
SEQ ID NO: 46 shows the amino acid sequence of L36E9VK-CK.
SEQ ID NO: 47 shows the amino acid sequence of L26B11VK-CK.
SEQ ID NO: 48 shows the amino acid sequence of R1B9VH-h1-CK-cross-IgG1m3-K.
SEQ ID NO: 49 shows the amino acid sequence of R1B9VK-h1-CH1-cross.
SEQ ID NO: 50 shows the amino acid sequence of R72B7VH-IgG1m3-HI1.
SEQ ID NO: 51 shows the amino acid sequence of the heavy chain variable region RIB9VH.
SEQ ID NO: 52 shows the amino acid sequence of the light chain variable region R1B9VK.
SEQ ID NO: 53 shows the amino acid sequence of the heavy chain variable region of the DP47-IgG1m3 antibody.
SEQ ID NO: 54 shows the amino acid sequence of the light chain variable region of the DP47-IgG1m3 antibody.
SEQ ID NO: 55 shows the nucleotide sequence of primer PmCGR.
SEQ ID NO: 56 shows the nucleotide sequence of primer PmCKR.

### DETAILED DESCRIPTION OF THE INVENTION

The inventors of the present application have made a thorough research on and a development of antibody drugs that bind human IL-36R and/or human IL-1R3, and have obtained novel bispecific antibodies and monoclonal antibodies that bind human IL-36R and/or human IL-1R3P by antibody engineering techniques. In various aspects of the present application, provided are novel bispecific and monoclonal antibodies that bind human IL-36R and/or human IL-1R3, polynucleotides encoding the bispecific or monoclonal antibodies, vectors comprising the polynucleotides, host cells comprising the polynucleotides or vectors, methods of preparing and purifying the bispecific or monoclonal antibodies, and medical and biological applications of the bispecific or monoclonal antibodies. A full-length bispecific antibody molecule or monoclonal antibody molecule can be constructed from the sequences of variable regions in the bispecific antibodies or monoclonal antibodies provided herein and used in clinical as a medicament for preventing or treating IL-36-mediated diseases.

Unless otherwise indicated, the inventions of the present application can be implemented using conventional molecular biology, microbiology, cell biology, biochemistry, and immunological techniques in the art.

Unless otherwise indicated, the terms used in the present application have the meanings commonly understood by those skilled in the art.

### DEFINITION

As used herein, the term "antibody" refers to an immunoglobulin molecule that is capable of specifically binding to a target via at least one antigen recognition site located in a variable region of the immunoglobulin molecule. Targets include, but are not limited to, carbohydrates, polynucleotides, lipids, and polypeptides, and the like. As used herein, an "antibody" includes not only an intact (*i.e.,* full-length) antibody, but also an binding fragment thereof (e.g., Fab, Fab', F(ab')₂, or Fv), a variant thereof, a fusion protein comprising portions of an antibody, a humanized antibody, a chimeric antibody, a bispecific antibody, a linear antibody, a single-chain variable, a VHH antibody, a multi-specific antibody (e.g., a bispecific antibody), and any other modified configurations of an immunoglobulin molecule comprising a desired specific antigen recognition site, including a glycosylated variant of an antibody, an amino acid sequence variant of an antibody, and a covalently modified antibody.

Typically, an intact or full-length antibody comprises two heavy chains and two light chains. Each heavy chain contains a heavy chain variable region (VH) and first, second and third constant regions (CH1, CH2 and CH3). Each light chain contains a light chain variable region (VL) and a constant region (CL). A full-length antibody may be of any type of antibody, such as an IgD, IgE, IgG, IgA, or IgM (or their subtypes) antibody, but not necessarily belongs to any particular type. Immunoglobulins can be assigned to different types depending on their amino acid sequences of the heavy chain constant domains. Generally, immunoglobulins have five main types, *i.e.,* IgA, IgD, IgE, IgG, and IgM, and some of these types can be further classified into subtypes (isotypes), such as IgG1, IgG2, IgG3, IgG4, IgA1, and IgA2. Heavy chain constant domains corresponding to individual immunoglobulin types are referred to as α, δ, ε, γ, and µ, respectively. Subunit structures and three-dimensional structures of different types of immunoglobulins are well known.

As used herein, the term "bispecific antibody" refers to an antibody that has binding capacities for two antigen epitopes. The two antigen epitopes may be on different antigens or on the same antigen. Bispecific antibodies may have a variety of structural configurations. For example, a bispecific antibody may consist of two Fc fragments and two binding moieties fused thereto, respectively (similar to a native antibody, except that the two arms bind a different antigen target or epitope), and the antigen-binding moieties may be single chain fragment variables (scFvs) or Fab fragments. When two non-overlapping epitopes of a given same antigen are targeted, two different binding portions of the bispecific antibody each bind to the N-terminus of one Fc fragment, and the antigen-binding portions of the two arms may be in a configuration from four combinations: scFv+Fab fragment, Fab fragment+scFv, scFv+scFv, and Fab fragment+Fab fragment. The Fc fragment may contain a mutation(s) that ensures heavy chain heteropolymerization, and KIH technique (knob-in-hole, KIH) is a strategy to address the heavy chain heteropolymerization. Generally, the KIH technique means that by modifying the amino acid sequence of the CH3 region to form a structure that facilitates pairing of the heterologous hemiantibodies to each other, the structure of the normal antibody can be maintained as much as possible while constituting the bispecific antibody. For guidance on the KIH technique, see, for example, "An efficient route to human bispecific IgG", A. Margaret Merchant et al., Nature Biotechnology, Volume 16, 1998, which is incorporated herein by reference in its entirety. In addition, the structure of the bispecific antibody may be an antibody that binds to the first epitope of the antigen (e.g., in the form of a native antibody) extending from the C-terminus of the CH3 region (which may be through a flexible linker) to the antigen-binding portion of the second epitope of the antigen.

As used herein, the terms "binding portion" or "binding fragment" are used interchangeably to refer to a portion or region of an intact antibody molecule responsible for binding to an antigen. The antigen-binding domain may comprise a heavy chain variant region (VH), a light chain variant region (VL), or both. Each of VH and VL typically contains three complementarity determining regions, CDR1, CDR2, and CDR3.

It is well known to those skilled in the art that complementarity determining regions (CDRs, usually including CDR1, CDR2 and CDR3) are the regions of a variable region that have mostly impact on the affinity and specificity of an antibody. The CDR sequences of VH or VL have two common definitions, *i.e.,* the Kabat definition and the Chothia definition (see, e.g., Kabat, "Sequences of Proteins of Immunological Interest", National Institutes of Health, Bethesda, Md. (1991); A1-Lazikani et al., J. Mol. Biol. 273:927-948 (1997); and Martin et al., Proc. Natl. Acad. Sci. USA 86:9268-9272 (1989)). For the sequences of the variable regions of a given antibody, the CDR sequences in VH and VL sequences can be determined according to the Kabat definition or the Chothia definition. In an embodiment of the present application, the CDR sequences are defined according to Kabat.

For the sequences of the variable regions of a given antibody, the CDR sequences in the sequences of variable regions can be determined in a variety of ways, for example, using online software Abysis (http://www.abysis.org/).

For typical antibodies, examples of an antigen-binding fragment include, but are not limited to, (1) an Fab fragment, which can be a monovalent fragment having a VL-CL chain and a VH-CH1 chain; (2) an F(ab')₂ fragment, which can be a divalent fragment having two Fab' fragments linked by a disulfide bridge of the hinge region (*i.e.,* a dimer of Fab'); (3) an Fv fragment having VL and VH domains in a single arm of an antibody; (4) a single-chain Fv (scFv), which can be a single polypeptide chain consisting of a VH domain and a VL domain via a polypeptide linker; and (5) (scFv)₂, which can comprise two VH domains linked by a peptide linker and two VL domains that are combined with the two VH domains via a disulfide bridge; and (6) VHH antibody forms.

In the configuration of a bispecific antibody, "binding moieties" include, but are not limited to, Fab fragment forms or single chain fragment variable (scFv) forms.

As used herein, the term "single chain fragment variable (scFv)" refers to an antibody of a single chain structure, generally constructed using genetic engineering techniques, comprising a polypeptide chain comprising a heavy chain variable region (VH) and a light chain variable region (VL). A flexible linker is typically designed between the heavy chain variable region and the light chain variable region so that the heavy chain variable region and the light chain variable region can be folded into the correct conformation capable of binding to the antigen.

As used herein, the term "Fab (fragment antigen binding) fragment", "Fab portion" or a similar term refers to an antibody fragment that is capable of binding to an antigen and that is produced after treatment of an intact antibody with papain, including an intact light chain (VL-CL), a heavy chain variable region, and a CH1 fragment (VH-CH1).

As used herein, the terms "first heavy chain constant region" and "second heavy chain constant region" both refer to "Fc fragment", "Fc domain", "Fc portion", or a similar term, meaning the portion of the antibody's heavy chain constant region, which includes the hinge region, CH2 domain, and CH3 domain of the heavy chain constant region, determined according to EU numbering for human IgG1 antibody.

As used herein, the term "monoclonal antibody" refers to an antibody from a population of substantially homogeneous antibodies, which means that the antibodies constituting the population are the same except for naturally occurring mutations which may occur in a small number of individual antibodies. Monoclonal antibodies described herein particularly include "chimeric" antibodies in which a portion of the heavy chain and/or the light chain is identical or homologous to a corresponding sequence in an antibody derived from a particular species or belonging to a particular antibody type or subtype, while the remaining portion of the heavy chain and/or the light chain is identical or homologous to a corresponding sequence in an antibody derived from another species or belonging to another antibody type or subtype; and also include fragments of such antibodies as long as they are capable of exhibiting the desired biological activity (see, U.S. Patent No. 4,816,567; and Morrison et al., Proc. Natl. Acad. Sci. USA 81: 6851-6855 (1984)).

As used herein, the term "specific binding" refers to a non-random binding reaction between two molecules, such as binding of an antibody to an epitope.

In a first aspect, the present application provides a bispecific antibody comprising a first antigen-binding fragment that binds to human IL-36R and a second antigen-binding fragment that binds to human IL-1R3.

In some embodiments of the first aspect, the bispecific antibody is capable of inhibiting an IL-36-mediated inflammatory response.

In some embodiments of the first aspect, the bispecific antibody is capable of inhibiting binding of IL-36α, IL-36β, and/or IL-36γ to IL-36R.

In some embodiments of the first aspect, the bispecific antibody is capable of inhibiting SEAP secretion by a cell.

In some embodiments of the first aspect, the bispecific antibody is capable of inhibiting IL-8 secretion by a cell.

In some embodiments of the first aspect, the first antigen-binding fragment comprises:
HCDR1 having the amino acid sequence SSWIH (SEQ ID NO: 1), HCDR2 having the amino acid sequence EINPGNVRSNYNENFRN (SEQ ID NO: 2), HCDR3 having the amino acid sequence INYGEPYFAS (SEQ ID NO: 3), LCDR1 having the amino acid sequence RASQSVIGSYLA (SEQ ID NO: 4), LCDR2 having the amino acid sequence SASKLAS (SEQ ID NO: 5), and LCDR3 having the amino acid sequence QQDTRYPIT (SEQ ID NO: 6); or
HCDR1 having the amino acid sequence SSWIH (SEQ ID NO: 1), HCDR2 having the amino acid sequence EINPGSGRSNYNENFQG (SEQ ID NO: 7), HCDR3 having the amino acid sequence VFYGEPYFPF (SEQ ID NO: 8), LCDR1 having the amino acid sequence RASQSVIYSYLA (SEQ ID NO: 9), LCDR2 having the amino acid sequence SASKRAS (SEQ ID NO: 10), and LCDR3 having the amino acid sequence QQHAGIPVT (SEQ ID NO: 11);
wherein the amino acid sequences of the HCDRs and the LCDRs are defined according to Kabat.

In some embodiments of the first aspect, the second antigen-binding fragment comprises:
HCDR1 having the amino acid sequence SYAMS (SEQ ID NO: 12), HCDR2 having the amino acid sequence EISRGGSHTYYPDTVTG (SEQ ID NO: 13), HCDR3 having the amino acid sequence SYGNYPFDY (SEQ ID NO: 14), LCDR1 having the amino acid sequence RASQSVIGSYLA (SEQ ID NO: 4), LCDR2 having the amino acid sequence SASKLAS (SEQ ID NO: 5), and LCDR3 having the amino acid sequence QQDTRYPIT (SEQ ID NO: 6); or
HCDR1 having the amino acid sequence SYAMS (SEQ ID NO: 12), HCDR2 having the amino acid sequence EISRGGSHTYYPDTVTG (SEQ ID NO: 13), HCDR3 having the amino acid sequence SYGNYPFDY (SEQ ID NO: 14), LCDR1 having the amino acid sequence SASSSVSYMY (SEQ ID NO: 15), LCDR2 having the amino acid sequence DTSNLAS (SEQ ID NO: 16), and LCDR3 having the amino acid sequence QQWSSYPLT (SEQ ID NO: 17);
wherein the amino acid sequences of the HCDRs and the LCDRs are defined according to Kabat.

In some embodiments of the first aspect, the first antigen-binding fragment comprises a heavy chain variable region having the amino acid sequence set forth in SEQ ID NO: 18, and a light chain variable region having the amino acid sequence set forth in SEQ ID NO: 19.

In some embodiments of the first aspect, the first antigen-binding fragment comprises a heavy chain variable region having the amino acid sequence set forth in SEQ ID NO: 20, and a light chain variable region having the amino acid sequence set forth in SEQ ID NO: 21.

In some embodiments of the first aspect, the second antigen-binding fragment comprises a heavy chain variable region having the amino acid sequence set forth in SEQ ID NO: 22, and a light chain variable region having the amino acid sequence set forth in SEQ ID NO: 19.

In some embodiments of the first aspect, the second antigen-binding fragment comprises a heavy chain variable region having the amino acid sequence set forth in SEQ ID NO: 22, and a light chain variable region having the amino acid sequence set forth in SEQ ID NO: 23.

In some embodiments of the first aspect, the first antigen-binding fragment comprises a heavy chain variable region set forth in SEQ ID NO: 18 and a light chain variable region set forth in SEQ ID NO: 19, and the second antigen-binding fragment comprises a heavy chain variable region set forth in SEQ ID NO: 22 and a light chain variable region set forth in SEQ ID NO: 19.

In some embodiments of the first aspect, the first antigen-binding fragment comprises a heavy chain variable region set forth in SEQ ID NO: 20 and a light chain variable region set forth in SEQ ID NO: 21, and the second antigen-binding fragment comprises a heavy chain variable region set forth in SEQ ID NO: 22 and a light chain variable region set forth in SEQ ID NO: 23.

In some embodiments of the first aspect, the first antigen-binding fragment comprises a heavy chain set forth in SEQ ID NO: 50 and a light chain set forth in SEQ ID NO: 46, and the second antigen-binding fragment comprises a heavy chain set forth in SEQ ID NO: 45 and a light chain set forth in SEQ ID NO: 46.

In some embodiments of the first aspect, the first antigen-binding fragment comprises a heavy chain set forth in SEQ ID NO: 44 and a light chain set forth in SEQ ID NO: 47, and the second antigen-binding fragment comprises a heavy chain set forth in SEQ ID NO: 48 and a light chain set forth in SEQ ID NO: 49.

In some embodiments of the first aspect, the bispecific antibody is an IgG1 antibody comprising a first heavy chain constant region and a second heavy chain constant region, wherein amino acids at positions 354 and 366 of the first heavy chain constant region are C and W, respectively, and amino acids at positions 349, 366, 368, and 407 of the second heavy chain constant region are C, S, A, and V, respectively; the amino acid positions of the antibody constant regions are determined according to EU numbering.

In some embodiments of the first aspect, the bispecific antibody is an IgG1 antibody comprising a first heavy chain constant region and a second heavy chain constant region, wherein amino acids at positions 234, 235, and 331 of the first heavy chain constant region and the second heavy chain constant region are F, E, and S, respectively; the amino acid positions of the antibody constant regions are determined according to EU numbering.

In some embodiments of the first aspect, the first antigen-binding fragment and the second antigen-binding fragment are independently selected from a single chain fragment variable (scFv) or a Fab fragment.

In some embodiments of the first aspect, the first antigen-binding fragment and the second antigen-binding fragment are both Fab fragments.

In some embodiments of the first aspect, the amino acid sequence of the heavy chain variable region of the first antigen-binding fragment differs from the amino acid sequence set forth in SEQ ID NO: 18 or 20 by about 1, 2, 3, 4, 5, 6, 7, 8, 9, or 10 amino acid substitutions, deletions, and/or additions.

In some embodiments of the first aspect, the amino acid sequence of the light chain variable region of the first antigen-binding fragment differs from the amino acid sequence set forth in SEQ ID NO: 19 or 21 by about 1, 2, 3, 4, 5, 6, 7, 8, 9, or 10 amino acid substitutions, deletions, and/or additions.

In some embodiments of the first aspect, the amino acid sequence of the heavy chain variable region of the first antigen-binding fragment has at least 90%, 91%, 92%, 93%, 94%, 95%, 96%, 97%, 98%, 99% or more homology to the amino acid sequence set forth in SEQ ID NO: 18 or 20.

In some embodiments of the first aspect, the amino acid sequence of the light chain variable region of the first antigen-binding fragment has at least 90%, 91%, 92%, 93%, 94%, 95%, 96%, 97%, 98%, 99% or more homology to the amino acid sequence set forth in SEQ ID NO: 19 or 21.

In some embodiments of the first aspect, the C-terminal or N-terminal region of the amino acid sequence set forth in SEQ ID NO: 18 or 20 may also be truncated by about 1, 2, 3, 4, 5, 6, 7, 8, 9, 10, 11, 12, 13, 14, 15, 20, 25, or more amino acids while still retaining a function similar to that of the heavy chain variable region of the first antigen-binding fragment.

In some embodiments of the first aspect, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 18 or 20, and the resulting amino acid sequence still retains a function similar to that of the heavy chain variable region of the first antigen-binding fragment.

In some embodiments of the first aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in SEQ ID NO: 18 or 20, provided that the altered amino acid sequence substantially retains a function similar to that of the heavy chain variable region of the first antigen-binding fragment.

In some embodiments of the first aspect, the C-terminal or N-terminal region of the amino acid sequence set forth in SEQ ID NO: 19 or 21 may also be truncated by about 1, 2, 3, 4, 5, 6, 7, 8, 9, 10, 11, 12, 13, 14, 15, 20, 25, or more amino acids while still retaining a function similar to that of the light chain variable region of the first antigen-binding fragment.

In some embodiments of the first aspect, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 19 or 21, and the resulting amino acid sequence still retains a function similar to that of the light chain variable region of the first antigen-binding fragment.

In some embodiments of the first aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in SEQ ID NO: 19 or 21, provided that the altered amino acid sequence substantially retains a function similar to that of the light chain variable region of the first antigen-binding fragment.

In some embodiments of the first aspect, the amino acid sequence of the heavy chain variable region of the second antigen-binding fragment differs from the amino acid sequence set forth in SEQ ID NO: 22 by about 1, 2, 3, 4, 5, 6, 7, 8, 9, or 10 amino acid substitutions, deletions, and/or additions.

In some embodiments of the first aspect, the amino acid sequence of the light chain variable region of the second antigen-binding fragment differs from the amino acid sequence set forth in SEQ ID NO: 19 or 23 by about 1, 2, 3, 4, 5, 6, 7, 8, 9, or 10 amino acid substitutions, deletions, and/or additions.

In some embodiments of the first aspect, the amino acid sequence of the heavy chain variable region of the second antigen-binding fragment has at least 90%, 91%, 92%, 93%, 94%, 95%, 96%, 97%, 98%, 99% or more homology to the amino acid sequence set forth in SEQ ID NO: 22.

In some embodiments of the first aspect, the amino acid sequence of the light chain variable region of the second antigen-binding fragment has at least 90%, 91%, 92%, 93%, 94%, 95%, 96%, 97%, 98%, 99% or more homology to the amino acid sequence set forth in SEQ ID NO: 19 or 23.

In some embodiments of the first aspect, the C-terminal or N-terminal region of the amino acid sequence set forth in SEQ ID NO: 22 may also be truncated by about 1, 2, 3, 4, 5, 6, 7, 8, 9, 10, 11, 12, 13, 14, 15, 20, 25 or more amino acids while still retaining a function similar to that of the heavy chain variable region of the second antigen-binding fragment.

In some embodiments of the first aspect, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 22, and the resulting amino acid sequence still retains a function similar to that of the heavy chain variable region of the second antigen-binding fragment.

In some embodiments of the first aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in SEQ ID NO: 22, provided that the altered amino acid sequence substantially retains a function similar to that of the heavy chain variable region of the second antigen-binding fragment.

In some embodiments of the first aspect, the C-terminal or N-terminal region of the amino acid sequence set forth in SEQ ID NO: 19 or 23 may also be truncated by about 1, 2, 3, 4, 5, 6, 7, 8, 9, 10, 11, 12, 13, 14, 15, 20, 25, or more amino acids while still retaining a function similar to that of the light chain variable region of the second antigen-binding fragment.

In some embodiments of the first aspect, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 19 or 23, and the resulting amino acid sequence still retains a function similar to that of the light chain variable region of the second antigen-binding fragment.

In some embodiments of the first aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in SEQ ID NO: 19 or 23, provided that the altered amino acid sequence substantially retains a function similar to that of the light chain variable region of the second antigen-binding fragment.

In a second aspect, the present application provides a monoclonal antibody that binds to human IL-36R, comprising:
HCDR1 set forth in SEQ ID NO: 1,
HCDR2 set forth in SEQ ID NO: 7,
HCDR3 set forth in SEQ ID NO: 8,
LCDR1 set forth in SEQ ID NO: 9,
LCDR2 set forth in SEQ ID NO: 10, and
LCDR3 set forth in SEQ ID NO: 11; or
HCDR1 set forth in SEQ ID NO: 1,
HCDR2 set forth in SEQ ID NO: 2,
HCDR3 set forth in SEQ ID NO: 3,
LCDR1 set forth in SEQ ID NO: 4,
LCDR2 set forth in SEQ ID NO: 5, and
LCDR3 set forth in SEQ ID NO: 6; or
HCDR1 set forth in SEQ ID NO: 1,
HCDR2 set forth in SEQ ID NO: 7,
HCDR3 set forth in SEQ ID NO: 8,
LCDR1 set forth in SEQ ID NO: 4,
LCDR2 set forth in SEQ ID NO: 5, and
LCDR3 set forth in SEQ ID NO: 6;
wherein the amino acid sequences of the HCDRs and the LCDRs are defined according to Kabat.

In some embodiments of the second aspect, the monoclonal antibody has a heavy chain variable region having the amino acid sequence set forth in SEQ ID NO: 20, and a light chain variable region having the amino acid sequence set forth in SEQ ID NO: 21.

In some embodiments of the second aspect, the antibody has a heavy chain variable region having the amino acid sequence set forth in SEQ ID NO: 18, and a light chain variable region having the amino acid sequence set forth in SEQ ID NO: 19.

In some embodiments of the second aspect, the antibody has a heavy chain variable region having the amino acid sequence set forth in SEQ ID NO: 20, and a light chain variable region having the amino acid sequence set forth in SEQ ID NO: 19.

In some embodiments of the second aspect, the amino acid sequence of the heavy chain variable region of the monoclonal antibody differs from the amino acid sequence set forth in SEQ ID NO: 18 or 20 by about 1, 2, 3, 4, 5, 6, 7, 8, 9, or 10 amino acid substitutions, deletions, and/or additions.

In some embodiments of the second aspect, the amino acid sequence of the light chain variable region of the monoclonal antibody differs from the amino acid sequence set forth in SEQ ID NO: 19 or 21 by about 1, 2, 3, 4, 5, 6, 7, 8, 9, or 10 amino acid substitutions, deletions, and/or additions.

In some embodiments of the second aspect, the amino acid sequence of the heavy chain variable region of the monoclonal antibody has at least 90%, 91%, 92%, 93%, 94%, 95%, 96%, 97%, 98%, 99% or more homology to the amino acid sequence set forth in SEQ ID NO: 18 or 20.

In some embodiments of the second aspect, the amino acid sequence of the light chain variable region of the monoclonal antibody has at least 90%, 91%, 92%, 93%, 94%, 95%, 96%, 97%, 98%, 99% or more homology to the amino acid sequence set forth in SEQ ID NO: 19 or 21.

In some embodiments of the second aspect, the C-terminal or N-terminal region of the amino acid sequence set forth in SEQ ID NO: 18 or 20 may also be truncated by about 1, 2, 3, 4, 5, 6, 7, 8, 9, 10, 11, 12, 13, 14, 15, 20, 25 or more amino acids while still retaining a function similar to that of the heavy chain variable region of the monoclonal antibody.

In some embodiments of the second aspect, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 18 or 20, and the resulting amino acid sequence still retains a function similar to that of the heavy chain variable region of the monoclonal antibody.

In some embodiments of the second aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in SEQ ID NO: 18 or 20, provided that the altered amino acid sequence substantially retains a function similar to that of the heavy chain variable region of the monoclonal antibody.

In some embodiments of the second aspect, the C-terminal or N-terminal region of the amino acid sequence set forth in SEQ ID NO: 19 or 21 may also be truncated by about 1, 2, 3, 4, 5, 6, 7, 8, 9, 10, 11, 12, 13, 14, 15, 20, 25 or more amino acids while still retaining a function similar to that of the light chain variable region of the monoclonal antibody.

In some embodiments of the second aspect, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 19 or 21, and the resulting amino acid sequence still retains a function similar to that of the light chain variable region of the monoclonal antibody.

In some embodiments of the second aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in SEQ ID NO: 19 or 21, provided that the altered amino acid sequence substantially retains a function similar to that of the light chain variable region of the monoclonal antibody.

In a third aspect, the present application provides a monoclonal antibody that binds to human IL-1R3, comprising
HCDR1 set forth in SEQ ID NO: 12,
HCDR2 set forth in SEQ ID NO: 13,
HCDR3 set forth in SEQ ID NO: 14,
LCDR1 set forth in SEQ ID NO: 15,
LCDR2 set forth in SEQ ID NO: 16, and
LCDR3 set forth in SEQ ID NO: 17;
wherein the amino acid sequences of the HCDRs and the LCDRs are defined according to Kabat.

In some embodiments of the third aspect, the monoclonal antibody has a heavy chain variable region having the amino acid sequence set forth in SEQ ID NO: 22, and a light chain variable region having the amino acid sequence set forth in SEQ ID NO: 23.

In some embodiments of the third aspect, the antibody has a heavy chain variable region having the amino acid sequence set forth in SEQ ID NO: 51, and a light chain variable region having the amino acid sequence set forth in SEQ ID NO: 52.

In some embodiments of the third aspect, the amino acid sequence of the heavy chain variable region of the monoclonal antibody differs from the amino acid sequence set forth in SEQ ID NO: 22 or 51 by about 1, 2, 3, 4, 5, 6, 7, 8, 9, or 10 amino acid substitutions, deletions, and/or additions.

In some embodiments of the third aspect, the amino acid sequence of the light chain variable region of the monoclonal antibody differs from the amino acid sequence set forth in SEQ ID NO: 23 or 52 by about 1, 2, 3, 4, 5, 6, 7, 8, 9, or 10 amino acid substitutions, deletions, and/or additions.

In some embodiments of the third aspect, the amino acid sequence of the heavy chain variable region of the monoclonal antibody has at least 90%, 91%, 92%, 93%, 94%, 95%, 96%, 97%, 98%, 99% or more homology to the amino acid sequence set forth in SEQ ID NO: 22 or 51.

In some embodiments of the third aspect, the amino acid sequence of the light chain variable region of the monoclonal antibody has at least 90%, 91%, 92%, 93%, 94%, 95%, 96%, 97%, 98%, 99% or more homology to the amino acid sequence set forth in SEQ ID NO: 23 or 52.

In some embodiments of the third aspect, the C-terminal or N-terminal region of the amino acid sequence set forth in SEQ ID NO: 22 or 51 may also be truncated by about 1, 2, 3, 4, 5, 6, 7, 8, 9, 10, 11, 12, 13, 14, 15, 20, 25 or more amino acids while still retaining a function similar to that of the heavy chain variable region of the monoclonal antibody.

In some embodiments of the third aspect, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 22 or 51, and the resulting amino acid sequence still retains a function similar to that of the heavy chain variable region of the monoclonal antibody.

In some embodiments of the third aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in SEQ ID NO: 22 or 51, provided that the altered amino acid sequence substantially retains a function similar to that of the heavy chain variable region of the monoclonal antibody.

In some embodiments of the third aspect, the C-terminal or N-terminal region of the amino acid sequence set forth in SEQ ID NO: 23 or 52 may also be truncated by about 1, 2, 3, 4, 5, 6, 7, 8, 9, 10, 11, 12, 13, 14, 15, 20, 25, or more amino acids while still retaining a function similar to that of the light chain variable region of the monoclonal antibody.

In some embodiments of the third aspect, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 23 or 52, and the resulting amino acid sequence still retains a function similar to that of the light chain variable region of the monoclonal antibody.

In some embodiments of the third aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in SEQ ID NO: 23 or 52, provided that the altered amino acid sequence substantially retains a function similar to that of the light chain variable region of the monoclonal antibody.

In a fourth aspect, the present application provides a pharmaceutical composition comprising the bispecific antibody of the first aspect, or the monoclonal antibody of the second or third aspect, and a pharmaceutically acceptable excipient, diluent, or carrier.

In some embodiments of the fourth aspect, the pharmaceutical composition is used to prevent or treat an IL-36-mediated disease.

In some embodiments of the fourth aspect, the IL-36-mediated disease is selected from the group consisting of psoriasis vulgaris, inflammatory bowel disease, renal injury and inflammation, pneumonia, arthritis, hidradenitis suppurative, generalized pustular psoriasis, and palmoplantar pustular psoriasis.

In some embodiments of the fourth aspect, the pharmaceutical composition can further comprise one or more of a lubricant, such as talc, magnesium stearate, and mineral oil; a wetting agent; an emulsifier; a suspending agent; a preservative such as benzoic acid, sorbic acid and calcium propionate; a sweetening agent and/or a flavoring agent.

In some embodiments of the fourth aspect, the pharmaceutical composition herein can be formulated as a tablet, a pill, a powder, a lozenge, an elixir, a suspension, an emulsion, a solution, a syrup, a suppository, a capsule, or the like.

In some embodiments of the fourth aspect, the pharmaceutical composition of the present application can be delivered using any physiologically acceptable administration route which includs, but is not limited to, oral administration, parenteral administration, nasal administration, rectal administration, intraperitoneal administration, intravascular injection, subcutaneous administration, transdermal administration, inhalation administration, or the like.

In some embodiments of the fourth aspect, a pharmaceutical composition for therapeutic uses can be formulated for storage in the form of a lyophilized formulation or an aqueous solution by mixing an agent with desired purity with a pharmaceutically acceptable carrier or excipient where appropriate.

In a fifth aspect, the present application provides the use of the bispecific antibody of the first aspect, the monoclonal antibody of the second or third aspect, or the pharmaceutical composition of the fourth aspect in the manufacture of a medicament for the prevention or treatment of an IL-36-mediated disease.

In some embodiments of the fifth aspect, the IL-36-mediated disease is selected from the group consisting of psoriasis vulgaris, inflammatory bowel disease, renal injury and inflammation, pneumonia, arthritis, hidradenitis suppurative, generalized pustular psoriasis, and palmoplantar pustular psoriasis.

In a sixth aspect, the present application provides a method of preventing or treating an IL-36-mediated disease, comprising administering to a subject in need thereof the bispecific antibody of the first aspect, the monoclonal antibody of the second or third aspect, or the pharmaceutical composition of the fourth aspect.

In some embodiments of the sixth aspect, the IL-36-mediated disease is selected from the group consisting of psoriasis vulgaris, inflammatory bowel disease, renal injury and inflammation, pneumonia, arthritis, hidradenitis suppurative, generalized pustular psoriasis, and palmoplantar pustular psoriasis.

In other aspects, the present application provides a nucleic acid molecule encoding the bispecific antibody of the first aspect, or the monoclonal antibody of the second or third aspect. In some embodiments, the nucleic acid molecule is operably linked to a regulatory sequence that can be recognized by a host cell transformed with the vector.

The present application also provides a vector comprising an isolated nucleic acid molecule encoding the bispecific antibody of the first aspect, or the monoclonal antibody of the second or third aspect, and a host cell comprising the nucleic acid molecule or vector. In other aspects, the application also provides a method of producing the bispecific antibody of the first aspect, or the monoclonal antibody of the second or third aspect. In some embodiments, the method of producing the bispecific antibody of the first aspect, or the monoclonal antibody of the second or third aspect, comprises culturing the host cell to facilitate expression of a nucleic acid molecule. In some embodiments, the method of producing the bispecific antibody of the first aspect, or the monoclonal antibody of the second or third aspect, further comprises recovering the bispecific antibody or monoclonal antibody from the culture medium for the host cell.

It is to be understood that the foregoing detailed description is intended only to enable those skilled in the art to have a better understanding of the present application and is not intended to cause limitations in any way. Various modifications and variations can be made to the described embodiments by those skilled in the art.

The following Examples are for purposes of illustration only and are not intended to limit the scope of the present application.

### Examples

### Example 1: Preparation of recombinant proteins

In the preparation and characterization of the IL-36R×IL-1R3 bispecific antibody, a variety of recombinant proteins were utilized, including the extracellular domain of human IL-36R (hIL-36R, SEQ ID NO: 24), the extracellular domain of monkey IL-36R (mfIL-36R, SEQ ID NO: 25), the extracellular domain of human IL-1R3 (hIL-1R3, SEQ ID NO: 26), the extracellular domain of monkey IL-1R3 (mfIL-1R3, SEQ ID NO: 27), the mature peptide of human IL-1 α (hIL-1α, SEQ ID NO: 28), the mature peptide of human IL-1β (hIL-1β, SEQ ID NO: 29), the mature peptide of human IL-33 (hIL-33, SEQ ID NO: 30), the mature peptide of human IL-36α (hIL-36α, SEQ ID NO: 31), the mature peptide of human IL-36β (hIL-36β, SEQ ID NO: 32) and the mature peptide of human IL-36γ (hIL-36γ, SEQ ID NO: 33). Addition of a His tag (His, SEQ ID NO: 34) or a Fc fragment of mouse IgG2a antibody (mFc, SEQ ID NO: 35) to the C-terminus of these recombinant proteins facilitated purification and functional identification of the recombinant proteins. In the preparation of a recombinant antibody, the heavy chain constant region of the antibody may be a human IgG1 subtype (SEQ ID NO: 36) or various mutants of a selected human IgG1 subtype, such as IgG1m3 (SEQ ID NO: 37), IgG1m3-HI (SEQ ID NO: 38) or IgG1m3-K (SEQ ID NO: 39); and the light chain constant region may be a human kappa subtype (SEQ ID NO: 40) or a human lambda subtype (SEQ ID NO: 41).

Genes (including the His tag or mFc) for the various recombinant proteins described above were designed and synthesized according to the amino acid sequences of the recombinant proteins in the Uniprot database. The synthesized genes for the various recombinant proteins were cloned into a suitable vector for eukaryotic expression (such as pcDNA3.1 from invitrogen Inc., etc) using conventional techniques in molecular biology. Plasmids for recombinant protein expression prepared were then transfected with liposomes (such as 293fectin from invitrogen Inc.) or other cationic transfection reagent (such as PEI, etc) into HEK293 cells (such as HEK293F from invitrogen Inc., etc), which were cultured in suspension under a serum-free condition for 3-4 days. The culture supernatant was then harvested by centrifugation or the like.

Recombinant proteins expressed as fusions with His tags were subjected to one-step purification of the recombinant proteins from the culture supernatant using a metal chelate affinity chromatography column (such as HisTrap FF from GE Inc., etc). Antibodies and recombinant proteins expressed as fusions with Fc were subjected to one-step purification using a ProteinA/G affinity chromatography column (such as Mabselect SURE from GE Inc., etc). The preservation buffer for the recombinant proteins was then replaced with PBS (pH7.0) or another suitable buffer using a desalting column (such as Hitrap desaulting from GE Inc., etc). Proteins were sterilized by filtration and then stored in aliquots at -20°C for later use.

### Example 2: Screening and affinity analysis of fully human IL-36R antibody

### 2.1 Screening of fully human Fab library

A fully human Fab phage library (see Chinese Patent Application No. 202210871809.6) was screened by a solid phase screening strategy (as for the experimental protocol, see, Phage Display: A Practical Approach, Clackson, T. (USA) and Lowman, H. B. (USA) (ed.), translated by Lan Ma et al., Chemical Industry Press, May 2008) using the recombinant hIL-36R-His prepared in Example 1 as an antigen. 3 rounds of screening were carried out by means of binding, elution, neutralization, infection and amplification. Finally, one monoclonal antibody R49E11VH-h3+L26B11VK (the amino acid sequence of R49E11VH-h3 is set forth in SEQ ID NO: 20; and the amino acid sequence of L26B 11 VK is set forth in SEQ ID NO: 21) specifically binding to human IL-36R was obtained.

R49E11VH-h3+L26B11VK was prepared as a whole IgG1m3 subtype antibody by conventional means in molecular biology. The heavy chain variable region (SEQ ID NO: 42) and the light chain variable region (SEQ ID NO: 43) of Spesolimab were synthesized with reference to U.S. Pat. No. US9023995B2, and Spesolimab was prepared as a whole IgG1m3 subtype antibody for a control study.

### 2.2 Affinity analysis of fully human monoclonal antibody

The affinity of the anti-human IL-36R monoclonal antibody was determined by the surface plasmon resonance technique using Biacore T200. Reagents and consumables such as an amino-coupling kit (BR-1000-50), a human antibody capture kit (BR-1008-39), a S Series CM5 chip (14100530) and 10×HBS-EP (BR100669) at pH7.4 were purchased from GE healthcare. The surface of the carboxylated CM5 chip was activated with 1-Ethyl-3-(3-dimethylaminopropyl) carbodiimide hydrochlorid (EDC) and N-Hydroxysuccinimide (NHS) according to the manual in the kit. An anti-human IgG (Fc) antibody (capture antibody) was diluted to 25µg/mL with 10mM sodium acetate (pH5.0), and then injected at a flow rate of 10µL/min to achieve a coupling amount of approximately up to 10,000 response units (RU). Followed by the injection of the capture antibody, 1M ethanolamine was injected to block unreacted groups. For the kinetics measurement, the anti-IL-36R monoclonal antibody was diluted to 1µg/mL, and injected at 10µL/min to ensure that approximately 200RU of the antibody was captured by the anti-human Fc antibody. hIL-36R-His was then set to a serial concentration gradient (e.g., 6.17nM, 18.5nM, 55.6nM, 166.7nM, and 500nM) and injected at 25°C from lower to higher concentrations at 30µL/min, with an association time of 90s and a dissociation time of 2,400s. A 3M MgCl₂ solution was injected at 10µL/min for 30s to regenerate the surface of the chip. The association rate (Kₐ) and dissociation rate (K_{d}) were calculated by fitting the binding and dissociation sensing grams with a 1:1 binding model using Biacore T200 evaluation software version 3.2.1. The dissociation equilibrium constant (K_{D}) was calculated as the ratio K_{d}/Kₐ. The fitting results are shown in Table 1.

**Table 1. Affinity constants for binding of IL-36R monoclonal antibody to hIL-36R-His**

| | Kₐ(M⁻¹s⁻¹) | K_{d}(_{S}⁻¹) | K_{D} (M) |
|---|---|---|---|
| R49E11VH-h3+L26B11VK | 2.282E+4 | 6.630E-5 | 2.905E-9 |
| Spesolimab | 2.883E+4 | 7.388E-5 | 2.562E-9 |

### 2.3 Analysis of biological activity of IL-36R monoclonal antibody based on HEK-Blue^{™} IL-36 cells

HEK-Blue^{™} IL-36 cells (Invivogen, cat. No. hkb-hil36r) were a reporter cell line developed by InvivoGen based on HEK293 cells. This cell line was stably transformed with human IL-36R, human IL-1R3, and SEAP (alkaline phosphatase) genes. When the cells were stimulated with IL-36α, IL-36β, and IL-36γ, the intracellular NF-κB signaling pathway was activated to induce expression of the SEAP reporter gene and secretion of SEAP into the supernatant. Concentration of SEAP in the supernatant may be quantitatively analyzed by a microplate reader at 630nm to reflect the activity of the stimulant.

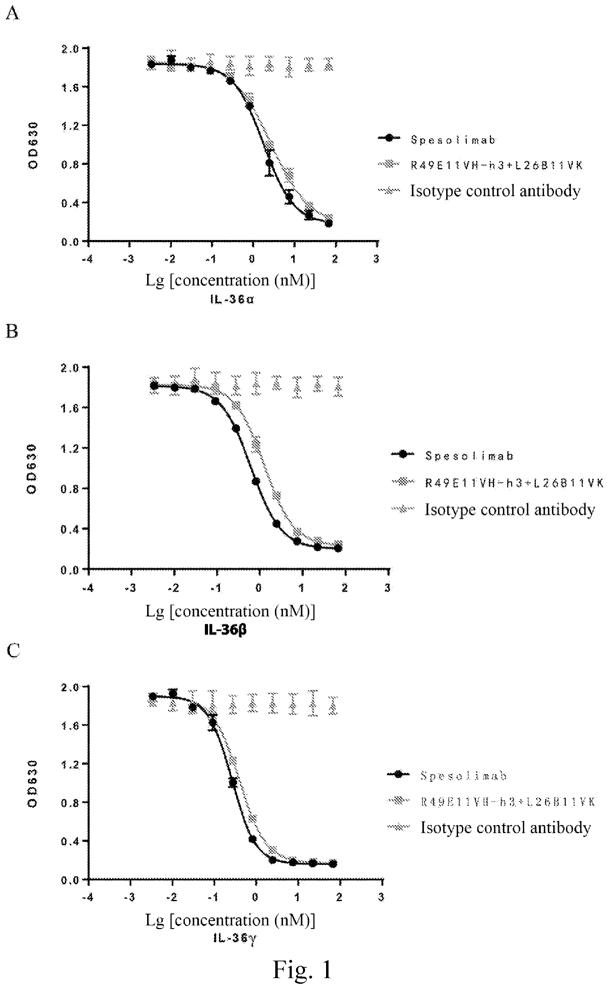

In this example, the ability of the IL-36R monoclonal antibody to inhibit IL-36α, IL-36β, and IL-36γ was evaluated with HEK-Blue^{™} IL-36 cells. HEK-Blue^{™} IL-36 cells were diluted to a single-cell suspension of 5 ×10⁵ cells/mL in HEK-Blue^{™} IL-36 cell maintenance medium (DMEM + 5% inactivated FBS + 1% penicillin-streptomycin (P/S)) and seeded at 100µL per well in a 96-well plate. The HEK-Blue^{™} IL-36 cells were then stimulated with IL-36α (0.2nM), IL-36β (0.01nM), or IL-36γ (0.05nM), followed by the addition of a serial concentration gradient (starting at 66.67nM, 3-fold gradient dilutions) of IL-36R monoclonal antibodies (R49E11VH-h3+L26B11VK and Spesolimab) and an isotype antibody (DP47-IgG1m3, prepared with reference to U.S. Patent Application US 20160200833A1, the amino acid sequences of the heavy chain variable region and the light chain variable region of which were set forth in SEQ ID NOs: 53 and 54, respectively) as negative control, cultured in a cell incubator (37±1°C, 5±1% CO₂) for 20h. 20µL of the supernatant was taken and transferred to a new 96-well ELISA plate. 180µL of a prepared solution of QUANTI-Blue^{™} (Invivogen, cat. No. rep-qbs) was added and incubated in a cell incubator (37±1°C, 5±1% CO₂) for 30min. Detection was performed using an absorption microplate reader at a wavelength of 630nm. The results showed that R49E11VH-h3+L26B11VK inhibited IL-36α, IL-36β and IL-36γ from stimulating HEK-Blue^{™} IL-36 cells, with activity comparable to Spesolimab (Figures 1A-1C).

### 2.4 Analysis of biological activity of IL-36R monoclonal antibody based on HaCAT cells

HaCAT was an immortalized human epidermal cell line, also known as an epidermal keratinocyte cell line, cultivated from keratinocytes and was an immortalized cell that retained the differentiation ability of keratinocytes. The IL-36 cytokines (IL-36α/IL-36β/IL-36γ) were able to stimulate IL-8 secretion by HaCAT cells.

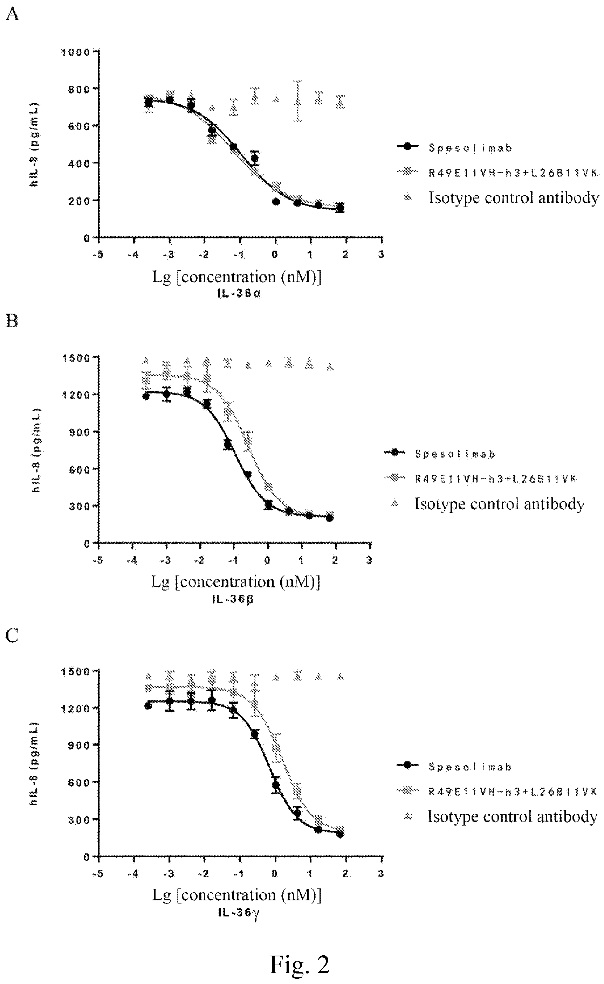

In this example, the ability of the IL-36R monoclonal antibody to inhibit IL-36α, IL-36β, and IL-36γ was evaluated with HaCAT cells. HaCAT cells were diluted to a single-cell suspension of 5×10⁵ cells/mL in a maintenance medium (MEM/EBSS + 5% inactivated FBS + 1% P/S) and seeded at 100 µL per well in a 96-well plate. The HaCAT cells were then stimulated with IL-36α (1nM), IL-36β (0.277nM) or IL-36γ (4.432nM), followed by the addition of a serial concentration gradient (starting at 66.67nM, 4-fold gradient dilutions) of anti-IL-36R monoclonal antibodies (R49E11VH-h3+L26B11VK, Spesolimab) and an isotype antibody (DP47-IgG1m3) as negative control were added, incubated in a cell incubator (37±1°C, 5±1% CO₂) for 20h. The supernatant was subjected to dilution and detection with a human IL-8 precoated ELISA kit (Dakewe Biotechnology LLC., cat. No. 1110802). The results showed that R49E11VH-h3+L26B11VK inhibited IL-36α, IL-36β and IL-36γ from stimulating hIL-8 secretion by HaCAT cells, with activity comparable to Spesolimab (Figures 2A-2C).

### Example 3: Preparation and affinity analysis of mouse IL-1R3 monoclonal antibody

### 3.1 Mouse immunization and preparation of antibody library

BALB/c mice of 6-8 weeks old were subjected to blood sampling from tail veins to retain baseline serum before immunization. For the first immunization, hIL-1R3-His fusion protein was emulsified with Freund's complete adjuvant, and each mouse was intraperitoneally injected with 50µg of the fusion protein. Booster immunizations were performed at intervals of two weeks. For the first and the third booster immunizations, the hIL-1R3-His fusion protein was emulsified with Freund's incomplete adjuvant, and each mouse was subjected to blood sampling by tail clipping before intraperitoneal injection with 50µg of the fusion protein. For the second and fourth booster immunizations, mfIL-1R3-His was emulsified with Freund's incomplete adjuvant, and 100µg of the fusion protein was injected intraperitoneally into each mouse. For the sixth immunization, hIL-1R3-His recombinant antigen as immunogen was used without an adjuvant, and 50µg of the fusion protein was intraperitoneally injected into each mouse. Mice were sacrificed 3 days after the rush immunization, and collected for splenocytes.

Mouse spleen lymphocytes were isolated using a mouse lymphocyte isolation solution (Dakewe Biotechnology LLC., CAT#DKW33-R0100). Total RNA was extracted from the isolated lymphocytes using a cellular total RNA extraction kit (Tiangen Biochemical Technology (Beijing) LLC., CAT#DP430). The extracted total RNA was used as a template to synthesize cDNAs of the heavy chain variable regions and the light chain variable regions with a first strand cDNA synthesis kit (Thermo scientific, CAT#K1621), respectively. Gene-specific primers were used as primers for reverse transcription. The primers annealed to regions located in the heavy chain constant regions and light chain constant regions of antibodies, respectively, specific sequences of which were PmCGR: TGCATTTGAACTCCTTGCC (SEQ ID NO: 55) and PmCKR: CCATCAATCTTCCACTTGAC (SEQ ID NO: 56), respectively. The synthesized cDNAs were immediately stored at -70°C for later use. Then, the cDNAs obtained by reverse transcription were used as templates to amplify VH and VK genes of the mouse antibodies by PCR with the primers synthesized according to reference ⁹. Overlapping extension PCR technology was used to construct genes for single chain fragment variables (scFvs). Finally, the prepared genes for the mouse single chain fragment variables were cloned into the vector pADSCFV-S (see Chinese Patent Application No. 201510097117.0) to construct a scFv library. The antibody library had a capacity of 1.0E8, and an accuracy of 70%.

### 3.2 Screening of mouse single chain fragment variable library

The above constructed phage library displaying the mouse single chain fragment variables was screened by a solid phase screening strategy (as for the experimental protocol, see, Phage Display: A Practical Approach, Clackson, T. (USA) and Lowman, H. B. (USA) (ed.), translated by Lan Ma et al., Chemical Industry Press, May 2008.5) using the HIL-1R3-His and mfIL-1R3-His prepared in Example 1 as antigens. 3 rounds of screening were carried out by means of binding, elution, neutralization, infection and amplification. Finally, a number of single chain fragment variables with varied sequences were obtained, among which R1B9 specifically binded to human IL-1R3 with the best effect.

### 3.3 Affinity analysis of mouse monoclonal antibody

The affinity of the mouse anti-human IL-1R3 monoclonal antibody was determined by the surface plasmon resonance technique using Biacore T200. Reagents and consumables such as an amino-coupling kit (BR-1000-50), a human antibody capture kit (BR-1008-39), a S Series CM5 chip (14100530) and 10×HBS-EP at pH7.4 (BR100669) were purchased from GE healthcare. The surface of the carboxylated CM5 chip was activated with 1-Ethyl-3-(3-dimethylaminopropyl) carbodiimide hydrochlorid (EDC) and N-Hydroxysuccinimide (NHS) according to the manual in the kit. An anti-human IgG (Fc) antibody (capture antibody) was diluted to 25µg/mL with 10mM sodium acetate (pH5.0), and then injected at a flow rate of 10µL/min to achieve a coupling amount of approximately up to 10,000 response units (RU). Followed by the injection of the capture antibody, 1M ethanolamine was injected to block unreacted groups. For the kinetics measurement, the anti-IL-36R monoclonal antibody was diluted to 1µg/mL, and injected at 10µL/min to ensure that approximately 100RU of the antibody was captured by the anti-human Fc antibody. hIL-36R-His was then set to a serial concentration gradient (e.g., 6.17nM, 18.5nM, 55.6nM, 166.7nM, and 500nM) and injected at 25°C from lower to higher concentrations at 30µL/min, with an association time of 90s and a dissociation time of 600-1,200s. A 3M MgCl₂ solution was injected at 10µL/min for 30s to regenerate the surface of the chip. The association rate (Kₐ) and dissociation rate (K_{d}) were calculated by fitting the binding and dissociation sensing grams with a 1:1 binding model using Biacore T200 evaluation software version 3.2.1. The dissociation equilibrium constant (K_{D}) was calculated as the ratio K_{d}/Kₐ. The fitting results are shown in Table 2.

**Table 2. Affinity constants for binding of mouse monoclonal antibody to hIL-1R3-His**

| | Kₐ(M⁻¹s⁻¹) | K_{d}(_{S}⁻¹) | K_{D}(M) |
|---|---|---|---|
| R1B9 | 1.032E+5 | 1.101E-4 | 1.067E-9 |

### 3.4 Identification of ability of mouse monoclonal antibody to recognize cell surface IL-1R3

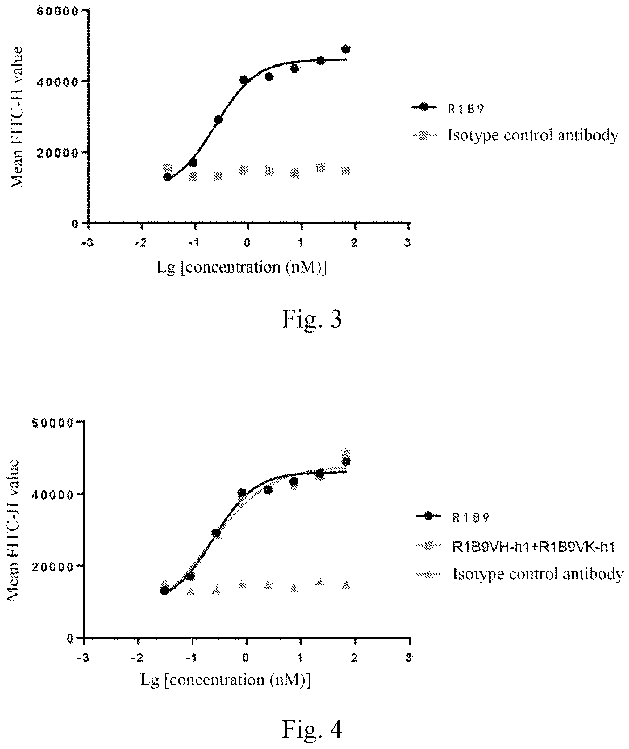

HaCAT cells at logarithmic growth phase were centrifuged, resuspended in a single cell suspension at 2×10⁶ cells/mL in PBS buffer containing 1% BSA, and plated at 100µL/wells in a 96-well V-bottom plate. 100µL of the mouse IL-1R3 monoclonal antibody R1B9 and an isotype antibody (DP47-IgG1m3) as negative control were diluted in a 3-fold gradient with 133.34nM as the starting concentration, added to the 96-well plate with the cells, incubated at 4°C for 1 hour, and then washed 3 times with 200µL of the PBS solution. Goat anti-human IgG-FITC (ZhongShanJinQiao, ZF-0308) was added at 100µL/well, incubated at 4°C for 30 minutes in the dark, washed three times with 200µL of the PBS solution, and then resuspended with 100µL of the PBS solution. The detection of FITC fluorescence was performed with a flow cytometer (ACEA, Novocyte). As shown in Figure 3, the mouse anti-human IL-1R3 monoclonal antibody R1B9 had excellent activity of binding to IL-1R3 on the surface of HaCAT cells.

### Example 4: Humanization and evaluation for activity of mouse IL-1R3 monoclonal antibody

### 4.1 Humanization of mouse monoclonal antibody R1B9

The mouse monoclonal antibody R1B9 was humanized to reduce its immunogenicity. A classic framework grafting strategy ¹⁰ was used for the humanization scheme. Nucleotide sequences encoding the heavy and light chain variable regions of R1B9 were compared to germline gene sequences of human antibodies in the IMGT database, respectively. Appropriate germline gene sequences were selected to provide framework region 1 to framework region 3 of the antibody (FR1+FR2+FR3), and an appropriate J region gene sequence was selected to provide framework region 4 (FR4). This template may be selected according to a variety of factors, such as relative total length of the antibody, CDR size, amino acid residues at the junction between the antibody framework regions (FR) and hypervariable regions (CDR), and overall homology of sequences. The selected template may be a mixture of multiple sequences or may be a consensus template in order to maintain the appropriate conformation of complementarity determining regions (CDRs) of the parent as much as possible. Finally, one humanized heavy chain mutant R1B9VH-h1 (SEQ ID NO: 22) and one humanized light chain mutant RIBO9VK-h1 (SEQ ID NO: 23) were obtained.

### 4.2 Affinity analysis of humanized antibody

Referring to Example 3.3, the affinity of humanized IL-1R3 antibody R1B9VH-h1+R1B9VK-h1 was analyzed using Biacore T200. Results are shown in Table 3.

**Table 3. Affinity constants for binding of humanized antibody to hIL-1R3-His**

| | Kₐ(M-¹s-¹ ) | K_{d}(S⁻¹) | K_{D} (M) |
|---|---|---|---|
| R1B9 | 7.499E+4 | 9.318E-5 | 1.243E-9 |
| R1B9VH-h1+R1B9VK-h1 | 9.916E+4 | 1.115E-4 | 1.125E-9 |

### 4.3 Identification of ability of humanized IL-1R3 antibody recognizing cell surface IL-1R3

Refer to Example 3.4 for specific implementation methods. As shown in Figure 4, the humanized antibody R1B9VH-h1+R1B9VK-h1 retained the binding activity of the parent R1B9.

### Example 5: Preparation and characterization of IL-36R×IL-1R3 Crossmab bispecific antibody

### 5.1 Preparation of Crossmab bispecific antibody

A Crossmab bispecific antibody was prepared from the parent antibodies R49E11VH-h3+L26B11VK and R1B9VH-h1+R1B9VK-h1. The nucleotide sequence encoding R49E11VH-h3 was cloned into a eukaryotic expression vector containing a nucleotide sequence encoding an IgG1 constant region IgG1m3-H1 having Hole mutations, the nucleotide sequence encoding R1B9VH-h1 was cloned into a eukaryotic expression vector containing a nucleotide sequence encoding an IgG1 constant region IgG1m3-K having Knob mutations, and the nucleotide sequences encoding L26B11VK and R1B9VK-h1 were cloned into eukaryotic expression vectors containing nucleotide sequences encoding a human light chain constant region CK. Then the nucleotide sequence encoding the heavy chain CH1 portion of the R1B9VH-h1 and the nucleotide sequence encoding the light chain CK portion of the RIB9VK-h1 were switched. The 4 eukaryotic expression vectors constructed for expressing R49E11VH-h3-IgG1m3-H1, L26B11VK-CK (SEQ IN NO: 47), R1B9VH-h1-CK-cross-IgG1m3-K (SEQ IN NO: 48) and R1B9VK-h1-CH1-cross (SEQ IN NO: 49) respectively were co-transfected into HEK293F cells with liposomes, which were cultured in suspension under a serum-free culture condition for 3-5 days. Then culture supernatant was collected by centrifugation or the like.

The bispecific antibody was purified from the culture supernatant using a ProteinA/G affinity chromatography column (such as Mabselect SURE from GE Inc., etc). Then the recombinant protein preservation buffer was replaced with PBS (pH 7.0) or another suitable buffer using a desalting column (such as Hitrap desaulting from GE Inc., etc). The desalted protein solution was subjected to purification by size exclusion chromatography (SEC) using Superdex200 (GE) to obtain the protein of interest. If necessary, the antibody sample may be sterilized by filtration and then stored in aliquots at -20°C for later use.

### 5.2 Affinity analysis of IL-36R×IL-1R3 Crossmab bispecific antibody

Referring to Examples 2.2 and 3.3, affinity analysis of the IL-36R×IL-1R3 Crossmab bispecific antibody was performed with Biacore T200. Results are shown in Table 4 and Table 5.

**Table 4. Affinity constants for binding of IL-36R×IL-1R3 Crossmab bispecific antibody to hIL-1R3-His**

| | Kₐ(M⁻¹s⁻¹ ) | K_{d}(_{S}⁻¹) | K_{D} (M) |
|---|---|---|---|
| Crossmab bispecific antibody (R1B9VH-h1+R1B9VK-h1-Fab-cross+R49E11VH-h3+L26B11VK-Fab) | 2.033E+5 | 1.547E-4 | 7.608E-10 |
| R1B9VH-h1+R1B9VK-h1 | 1.061E+5 | 1.534E-4 | 1.446E-9 |

**Table 5. Affinity constants for binding of IL-36R×IL-1R3 Crossmab bispecific antibody to human hIL-36R-His**

| | Kₐ(M-1s-1 ) | Kₐ(s-1) | K_{D} (M) |
|---|---|---|---|
| Crossmab bispecific antibody (R1B9VH-h1+R1B9VK-h1-Fab-cross+R49E11VH-h3+L26B11VK-Fab) | 6.537E+4 | 4.372E-5 | 6.688E-10 |
| R49E11VH-h3+L26B11VK | 3.886E+4 | 6.833E-5 | 1.758E-9 |

### 5.3 Analysis of biological activity of IL-36R×IL-1R3 Crossmab bispecific antibody based on HaCAT cells

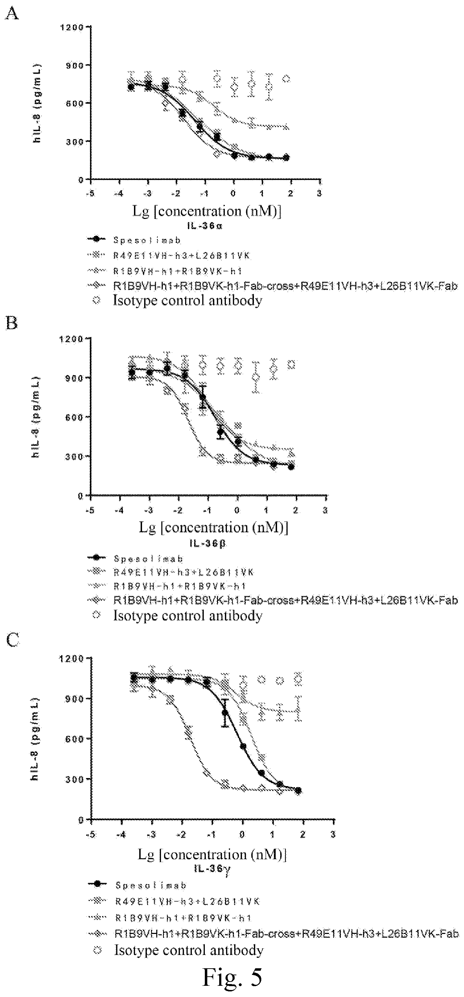

Refer to Example 2.4 for specific implementation methods. Results are shown in Figures 5A-5C and Table 6. The Crossmab bispecific antibody (R1B9VH-h1+R1B9VK-h1-Fab-cross+R49E11VH-h3+L26B11VK-Fab) showed a synergy compared to the R49E11VH-h3+L26B11VK monoclonal antibody. According to IC₅₀ values, the activity in the IL-36α direction was increased by about 3-fold, the activity in the IL-36β direction was increased by about 13-fold, and the activity in the IL-36γ direction was increased by about 103-fold.

**Table 6. IC₅₀ values of IL-36R×IL-1R3 Crossmab bispecific antibody for inhibiting IL-36 cytokines from inducing hIL-8 secretion by HaCAT cells**

| | IC₅₀(nM) | | |
|---|---|---|---|
| | IL-36α | IL-36β | IL-36γ |
| Spesolimab | 0.04329 | 0.1717 | 0.6417 |
| R49E11VH-h3+L26B11VK | 0.05333 | 0.2764 | 1.902 |
| R1B9VH-hl+R1B9VK-h1 | 0.1954 | 0.09611 | 0.6079 |
| Crossmab bispecific antibody (R1B9VH-h1+R1B9VK-h1-Fab-cross+R49E11VH-h3+L26B11VK-Fab) | 0.0183 | 0.02114 | 0.01842 |
| Isotype control antibody (DP47-IgG1m3) | / | / | / |

| | | | |
|---|---|---|---|
| Note: "/" indicates no activity. | | | |

### Example 6: Affinity maturation of IL36R×IL-1R3 common light chain bispecific antibody

### 6.1 Screening of common light chain

Based on a two-vector phage display system (see Chinese Patent Application No. 201510097117.0 for the experimental protocol) and the heavy chain R49E11VH-h3 of the anti-human IL-36R monoclonal antibody R49E11VH-h3+L26B11 VK, an original light chain library was subjected to 2 rounds of screening and enrichment using hIL-36R-His as the antigen for screening. The hIL-36R-His-enriched light chain library was then subjected to 2 rounds of screening and enrichment based on the heavy chain R1B9VH-h1 of the anti-human IL-1R3 monoclonal antibody R1B9VH-h1+R1B9VK-h1 using hIL-1R3-His as the antigen for screening. Finally, the light chain was obtained and identified. The common light chain variable region L36E9VK (SEQ ID NO: 19) was finally obtained.

### 6.2 Preparation of common light chain bispecific antibody

A bispecific antibody was prepared based on the common light chain variable region L36E9VK. The nucleotide sequence encoding R49E11VH-h3 was cloned into a eukaryotic expression vector containing the nucleotide sequence encoding the IgG1 constant region IgG1m3-H1 having Hole mutations, the nucleotide sequence encoding R1B9VH-h1 was cloned into a eukaryotic expression vector containing the nucleotide sequence encoding the IgG1 constant region IgG1m3-K having Knob mutations, and the nucleotide sequence encoding the common light chain L36E9VK was cloned into a eukaryotic expression vector containing the nucleotide sequence encoding the human light chain constant region CK. The 3 eukaryotic expression vectors constructed for expressing R49E11VH-h3-IgG1m3-H1 (SEQ ID NO: 44), R1B9VH-h1-IgG1m3-K (SEQ ID NO: 45) and L36E9VK-CK (SEQ ID NO: 46) respectively were co-transfected into HEK293F cells with liposomes, which were cultured in suspension under a serum-free culture condition for 3-5 days. Then culture supernatant was collected by centrifugation or the like.

The bispecific antibody was purified from the culture supernatant using a ProteinA/G affinity chromatography column (such as Mabselect SURE from GE Inc., etc). Then the recombinant protein preservation buffer was replaced with PBS (pH 7.0) or another suitable buffer using a desalting column (such as Hitrap desaulting from GE Inc., etc). The desalted protein solution was subjected to purification by size exclusion chromatography (SEC) using Superdex200 (GE) to obtain the protein of interest. If necessary, the antibody sample may be sterilized by filtration and then stored in aliquots at -20°C for later use.

### 6.3 Scheme for affinity maturation

In order to increase the binding capacity of the bispecific antibody in the IL-36R direction and to increase the activity of the IL-36R×IL-1R3 bispecific antibody to block the IL-36 cytokines, in vitro affinity maturation was performed on the R49E11VH-h3 heavy chain variable region. A library of CDR23 mutants based on R49E11VH-h3 was constructed by introducing mutations into CDR2 and CDR3 of the R49E11VH-h3 heavy chain variable region using conventional means in molecular biology. The scheme for mutagenesis was designed as shown in Table 7, with a constructed library capacity of 1.4E8 and an accuracy of 70%.

**Table 7. Scheme for designing library of R49E11VH-h3-CDR23 mutants**

| original amino acid (Kabat number) | Designed amino acid | Degenerate codon |
|---|---|---|
| 53G | N, S, G or D | RRT |
| 54S | N, S, G or D | RRT |
| 55G | G, V, A or D | GNC |
| 56R | R, S or G | VGC |
| 57S | S or T | AST |
| 58N | N or D | RAT |
| 64Q | Q or R | CRA |
| 65G | S, N, D or G | RRC |
| 66R | R or K | ARA |
| 95V | L, I or V | VTC |
| 96F | F, Y, I or N | WWT |
| 97Y | F, S or Y | THT |
| 98G | G, A or D | GVC |
| 99E | D or E | GAM |
| 100P | P or A | SCA |
| (100A)Y | F, S or Y | THT |
| (100B)F | F, Y, I or N | WWT |
| 101P | P or A | SCA |
| 102F | F, S or Y | THT |

### 6.4 Screening of library of mutants for affinity maturation

Based on a two-vector phage display system (see Example 5 in Chinese Patent Application No. 201510097117.0 for the experimental protocol), the constructed library of R49E11VH-h3-CDR23 mutants was subjected to three rounds of screening and enrichment by using the hIL36R-His antigen, with the light chain L36E9VK retaining unchanged. Finally, one heavy chain variable region mutant R72B7 (SEQ ID NO: 18) with increased affinity was obtained.

Referring to Example 1 and Example 5, a monoclonal antibody and a bispecific antibody molecule were prepared based on R72B7.

### 6.5 Affinity analysis of mutants

Referring to Examples 2.2 and 3.3, affinity of an IL-36R×IL-1R3 Crossmab bispecific antibody was analyzed using Biacore T200. Results are shown in Table 8 and Table 9.

**Table 8. Affinity constants for binding of IL-36R×IL-1R3 common light chain bispecific antibody to hIL-36R-His**

| | Kₐ(M⁻¹s⁻1) | K_{d}(_{S}⁻¹) | K_{D}(M) |
|---|---|---|---|
| Common light chain bispecific antibody (R72B7VH/RIBOVH-h1+L36E9VK) | 3.072E+4 | 3.979E-5 | 1.295E-9 |
| R72B7VH+L36E9VK | 3.279E+4 | 5.400E-5 | 1.647E-9 |
| R49E11VH-h3+L36E9VK | 8.686E+3 | 9.944E-5 | 1.145E-8 |

**Table 9. Affinity constants for binding of IL-36R×IL-1R3 bispecific antibody to hIL-1R3-His**

| | Kₐ(M⁻¹s⁻¹ ) | K_{d}(ₛ⁻¹) | K_{D}(M) |
|---|---|---|---|
| Common light chain bispecific antibody (R72B7VH/R1B9VH-h1+L36E9VK) | 3.835E+4 | 5.532E-5 | 1.443E-9 |
| R1B9VH-h1+R1B9VK-h1 | 1.061E+5 | 1.534E-4 | 1.446E-9 |

### 6.6 Analysis of biological activity of affinity-matured common light chain bispecific antibody based on HaCAT cells

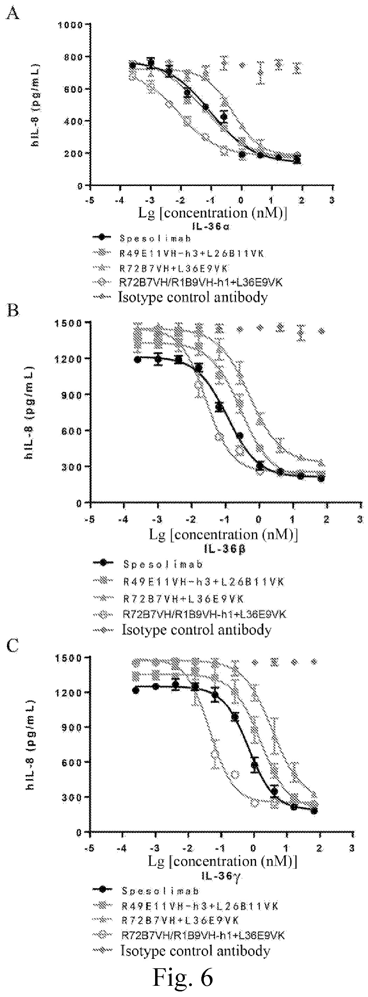

Refer to Example 2.4 for specific implementation methods. Results are shown in Figures 6A-6C and Table 10. The affinity-matured common light chain bispecific antibody (R72B7VH/R1B9VH-h1+L36E9VK) showed a significant synergy compared to the R72B7VH+L36E9VK monoclonal antibody. According to IC₅₀ values, the activity in IL-36α direction was increased by about 12-fold, the activity in the IL-36β direction was increased by about 4-fold, and the activity in the IL-36γ direction was increased by about 15-fold compared to Spesolimab.

**Table 10. IC₅₀ values of IL-36R×IL-1R3 common light chain bispecific antibody for inhibiting IL-36 from inducing hIL-8 secretion by HaCAT cells**

| | IC₅₀(nM) | | |
|---|---|---|---|
| | IL-36α | IL-36β | IL-36γ |
| Spesolimab | 0.09389 | 0.1142 | 0.6817 |
| R49E11VH-h3+L26B11VK | 0.0559 | 0.2803 | 1.576 |
| R72B7VH+L36E9VK | 0.5081 | 0.5113 | 3.652 |
| Common light chain bispecific antibody (R72B7VH/R1B9VH-h1+L36E9VK) | 0.007972 | 0.02697 | 0.04528 |
| Isotype control antibody (DP47-IgG1m3) | / | / | / |
| Note: "/" indicates no activity. | | | |

### 6.7 Analysis of biological activity of affinity-matured common light chain bispecific antibody based on human monocytes

### 6.7.1 Isolation of human peripheral blood mononuclear cells (PBMCs)

Blood was collected from normal volunteers (50mL each), all of whom had signed informed consent.

Inclusion criteria for volunteers were:
1. Age greater than 18 years;
2. No HIV, HBV infection;
3. Normal results of routine blood test;
4. Non-pregnant or lactating women.

PBMCs were isolated from whole blood cells from the volunteers by Ficoll density gradient centrifugation and cultured in 1640 medium.

### 6.7.2 Analysis of biological activity of affinity-matured common light chain bispecific antibody based on human monocytes

Peripheral blood mononuclear cells (PBMCs) refered to cells in the peripheral blood with each cell having a single nucleus, including lymphocytes, monocytes, dendritic cells, and other cells in small quantity. IL-36 cytokines (IL-36α/IL-36β/IL-36γ) were able to stimulate hIL-8 secretion by human monocytes.

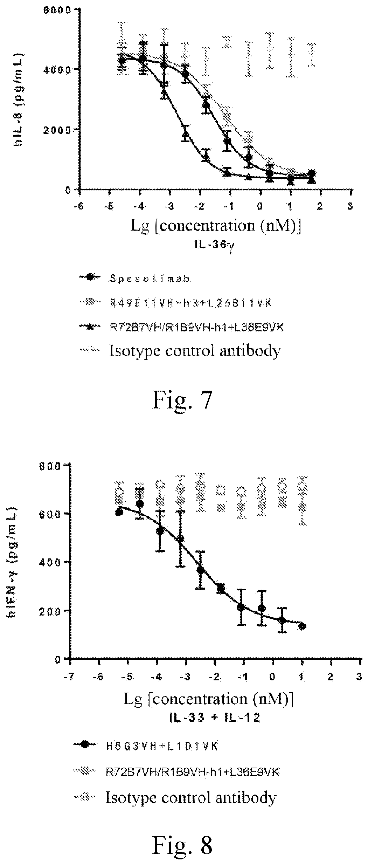

In this example, the ability of an IL-36R monoclonal antibody and an IL-36R×IL-1R3 bispecific antibody to inhibit IL-36γ was evaluated with human monocytes. PBMCs were isolated from peripheral blood of healthy individuals. CD14+ monocytes were obtained based on positive selection by magnetic beads (anti-human CD14 magnetic particles-DM, BD Bioscience, cat. No. 557769). The CD14+ monocytes were diluted with a monocyte maintenance medium (1640+10% inactivated FBS+1%P/S) to a single cell suspension of 5×10⁵ cells/mL, and seeded at 100µL/well into a 96-well plate. The CD14+ monocytes were stimulated with a certain concentration of IL-36γ (16.62nM), and added with test antibodies (R72B7VH/R1B9VH-h1+L36E9VK, R49E11VH-h3+L26B11VK, and Spesolimab) and an isotype antibody (DP47-IgG1m3) as negative control at a serial concentration gradient (50nM as the starting concentration, 5-fold gradient dilutions). After 48h incubation in a cell incubator, the supernatant was diluted and then detected with the human IL-8 precoated ELISA kit (Dakewe Biotechnology LLC., cat. No. 1110802). Results are shown in Figures 7 and Table 11. The affinity-matured common light chain bispecific antibody R72B7VH/R1B9VH-h1+L36E9VK showed a significant synergy compared to the R49E11VH-h3+L26B11VK monoclonal antibody. According to IC₅₀ values, the activity in the IL-36γ direction increased by approximately 17-fold compared to Spesolimab.

**Table 11. IC₅₀ values of affinity-matured common light chain bispecific antibody for inhibiting IL-36γ-induced hIL-8 secretion by human monocytes**

| | IC₅₀(nM) |
|---|---|
| | IL-36γ |
| Spesolimab | 0.0288 |
| R49E11VH-h3+L26B11VK | 0.06773 |
| Common light chain bispecific antibody (R72B7VH/R1B9VH-h1+L36E9VK) | 0.001714 |
| Isotype control antibody | / |

| | |
|---|---|
| Note: "/" indicates no activity. | |

### Example 7: Analysis of effect of IL-36R×IL-1R3 common light chain bispecific antibody on activity of IL-33 signaling pathway

IL-33 or IL-12 alone was unable to activate NK cells, but IL-12 was capable of significantly increasing the expression of ST2 on the surface of NK cells. When freshly isolated NK cells were stimulated with IL-33 and IL-12, IL-33 was able to bind to ST2 on the surface of the NK cells, which enabled activation of the NK cells to release a substantial amount of IFN-γ. By detecting the effect of an anti-ST2 antibody and an anti-IL-1R3 antibody on the level of IFN-γ expression, the evaluation of the activities of the antibodies was enabled.

PBMCs were isolated from peripheral blood of healthy individuals (referrs to Example 6.5.1 for the specific implementation method), diluted with a PBMC maintenance medium (1640+10% inactivated FBS+1%P/S) into a single cell suspension of 1×10⁶ cells/mL, seeded into a 96-well plate at 100µL/well, stimulated with certain concentrations of IL-12 (175pM) and IL-33 (1110pM), added with test antibodies (R72B7VH/R1B9VH-h1+L36E9VK and an anti-ST2 monoclonal antibody H5G3VH+L1D1VK (see Chinese Patent Application No. CN110357963A)) and an isotype antibody (DP47-IgG1m3) as negative control at a serial concentration gradient (10nM as the starting concentration, 5-fold gradient dilutions), and incubated in a cell incubator (37±1°C, 5±1% CO₂) for 24 hours. The supernatant was subjected to dilution and detection with an interferon gamma-matched ELISA antibody pair (Sino Biological, cat. No. SEKA11725). Results are shown in Figure 8. R72B7VH/R1B9VH-h1+L36E9VK was unable to inhibit IL-33 and IL-12 from stimulating human hIFN-γ release by NK cells.

### Example 8: Analysis of effect of IL-36R×IL-1R3 common light chain bispecific antibody on activity of IL-1 signaling pathway

MRC-5 was a human lung fibroblast, and IL-1α and IL-1β were able to stimulate IL-8 secretion by MRC-5 cells. By detecting the effect of an anti-IL-1R1 antibody and an anti-IL-1R3 antibody on the level of IL-8 expression, the evaluation of the activities of the antibodies was enabled.

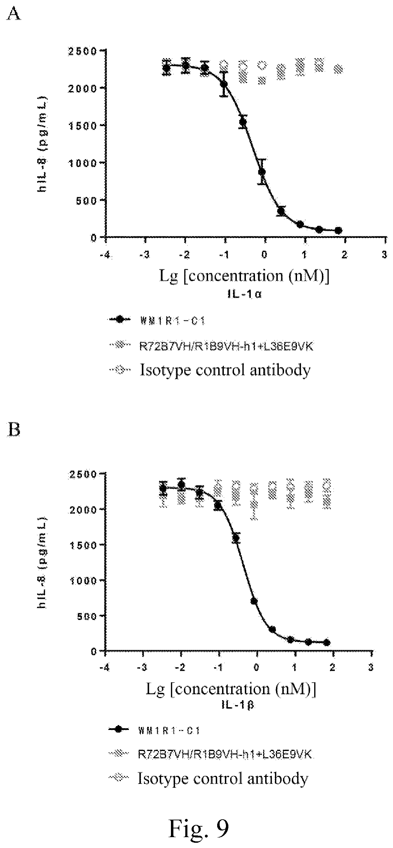

MRC-5 was diluted with an MRC-5 cell maintenance medium (MEM/EBSS+5% inactivated FBS+1% P/S) into a single cell suspension at 1×10⁵ cells/mL, and seeded at 100µL/well into a 96-well plate. MRC-5 cells were stimulated with a certain concentration of IL-1α (50pM) or IL-1β (5pM), and added with test antibodies (R72B7VH/R1B9VH-h1+L36E9VK and an anti-IL1R1 monoclonal antibody WM1R1-C1 (see 15C4 in U.S. Patent Application US8236559B2)) and an isotype antibody (DP47-IgG1m3) as negative control at a serial concentration gradient (66.67nM as the starting concentration, 3-fold gradient dilutions). After 20h incubation in a cell incubator (37±1°C, 5±1% CO₂), the supernatant was subjected to dilution and detection with the human IL-8 pre-coated ELISA kit (Dakewe Biotechnology LLC., cat. No. 1110802). Results are as shown in Figures 9A and 9B. R72B7/R1B9VH-h1+L36E9VK was unable to inhibit IL-1α or IL-1β from stimulating hIL-8 release by MRC-5 cells.

Exemplary embodiments of the inventions in the present application have been described hereinabove. However, those skilled in the art can modify or amend the exemplary embodiments described herein without departing from the spirit and scope of the present application, and variations or equivalents derived therefrom also fall within the scope of the present application.

### References

1. Gresnigt MS, van de Veerdonk FL. Biology of IL-36 cytokines and their role in disease. Semin Immunol. 2013 Dec 15;25(6):458-65.
2. Queen D, Ediriweera C, Liu L. Function and Regulation of IL-36 Signaling in Inflammatory Diseases and Cancer Development. Front Cell Dev Biol. 2019 Dec 4;7:317.
3. Zhou L, Todorovic V. Interleukin-36: Structure, Signaling and Function. Adv Exp Med Biol. 2021;21:191-210.
4. Neurath MF. IL-36 in chronic inflammation and cancer. Cytokine Growth Factor Rev. 2020 Oct;55:70-79.
5. Bachelez H, Choon SE, Marrakchi S, Burden AD, Tsai TF, Morita A, Turki H, Hall DB, Shear M, Baum P, Padula SJ, Thoma C. Inhibition of the Interleukin-36 Pathway for the Treatment of Generalized Pustular Psoriasis. N Engl J Med. 2019 Mar 7;380(10):981-983.
6. Bachelez H, Choon SE, Marrakchi S, Burden AD, Tsai TF, Morita A, Navarini AA, Zheng M, Xu J, Turki H, Anadkat MJ, Rajeswari S, Hua H, Vulcu SD, Hall D, Tetzlaff K, Thoma C, Lebwohl MG; Effisayil 1 Trial Investigators. Trial of Spesolimab for Generalized Pustular Psoriasis. N Engl J Med. 2021 Dec 23;385(26):2431-2440.
7. Mrowietz U, Burden AD, Pinter A, Reich K, Schäkel K, Baum P, Datsenko Y, Deng H, Padula SJ, Thoma C, Bissonnette R. Spesolimab, an Anti-Interleukin-36 Receptor Antibody, in Patients with Palmoplantar Pustulosis: Results of a Phase IIa, Multicenter, Double-Blind, Randomized, Placebo-Controlled Pilot Study. Dermatol Ther (Heidelb). 2021 Apr;11(2):571-585.
8. Navarini AA, Burden AD, Capon F, Mrowietz U, Puig L, Köks S, Kingo K, Smith C, Barker JN; ERASPEN Network. European consensus statement on phenotypes of pustular psoriasis. J Eur Acad Dermatol Venereol. 2017 Nov;31(11):1792-1799.
9. Krebber A, Bornhauser S, Burmester J, Honegger A, Willuda J, Bosshard HR, Plückthun A. Reliable cloning of functional antibody variable domains from hybridomas and spleen cell repertoires employing a reengineered phage display system. J Immunol Methods. 1997 Feb 14;201(1):35-55.
10. Tan P, Mitchell DA, Buss TN, Holmes MA, Anasetti C, Foote J. "Superhumanized" antibodies: reduction of immunogenic potential by complementarity-determining region grafting with human germline sequences: application to an anti-CD28. J Immunol. 2002 Jul 15; 169(2): 1119-25.

### Sequences

**SEQ ID NO:1**
   SSWIH
**SEQ ID NO:2**
   EINPGNVRSNYNENFRN
**SEQ ID NO:3**
   INYGEPYFAS
**SEQ ID NO:4**
   RASQSVIGSYLA
**SEQ ID NO:5**
   SASKLAS
**SEQ ID NO:6**
   QQDTRYPIT
**SEQ ID NO:7**
   EINPGSGRSNYNENFQG
**SEQ ID NO:8**
   VFYGEPYFPF
**SEQ ID NO:9**
   RASQSVIYSYLA
**SEQ ID NO:10**
   SASKRAS
**SEQ ID NO:11**
   QQHAGIPVT
**SEQ ID NO:12**
   SYAMS
**SEQ ID NO:13**
   EISRGGSHTYYPDTVTG
**SEQ ID NO:14**
   SYGNYPFDY
**SEQ ID NO:15**
   SASSSVSYMY
**SEQ ID NO:16**
   DTSNLAS
**SEQ ID NO:17**
   QQWSSYPLT
**SEQ ID NO:18** 
QVQLVQSGAEVKKPGASVKVSCKASGYSFTSSWIHWVKQAPGQGLEWMGEINPGNVR
SNYNENFRNKVTMTVDTSISTAYMELSRLRSDDTAVYYCTVINYGEPYFASWGQGTLV
TVSS
**SEQ ID NO:19** 
EIVLTQSPGTLSLSPGERATLSCRASQSVIGSYLAWYQQKPGQAPRLLIYSASKLASGIPD
RFSGSGSGTDFTLTISRLEPEDFAVYYCQQDTRYPITFGQGTKVEIK
**SEQ ID NO:20** 
QVQLVQSGAEVKKPGASVKVSCKASGYSFTSSWIHWVRQAPGQGLEWMGEINPGSGRS
NYNENFQGRVTMTVDTSISTAYMELSRLRSDDTAVYYCTVVFYGEPYFPFWGQGTLVT
VSS
**SEQ ID NO:21** 
EIVLTQSPGTLSLSPGERATLSCRASQSVIYSYLAWYQQKPGQAPRLLIYSASKRASGIPD
RFSGSGSGTDFTLTISRLEPEDFAVYYCQQHAGIPVTFGQGTKVEIK
**SEQ ID NO:22** 
EVQLVESGGGLVQPGGSLRLSCAASGFTFSSYAMSWVRQAPGKGLVWVAEISRGGSHT
YYPDTVTGRFTISRDNAKNTLYLQMNSLRAEDTAVYYCARSYGNYPFDYWGQGTTVT
VSS
**SEQ ID NO:23** 
EIVLTQSPATLSLSPGERATLSCSASSSVSYMYWYQQKPGQAPRLLIYDTSNLASGIPARFS
GSGSGTDFTLTISSLEPEDFAVYYCQQWSSYPLTFGQGTKLEIK
**SEQ ID NO:24** 
DGCKDIFMKNEILSASQPFAFNCTFPPITSGEVSVTWYKNSSKIPVSKIIQSRIHQDETWIL
FLPMEWGDSGVYQCVIKGRDSCHRIHVNLTVFEKHWCDTSIGGLPNLSDEYKQILHLGK
DDSLTCHLHFPKSCVLGPIKWYKDCNEIKGERFTVLETRLLVSNVSAEDRGNYACQAIL
THSGKQYEVLNGITVSITERAGYGGSVPKIIYPKNHSIEVQLGTTLIVDCNVTDTKDNTNL
RCWRVNNTLVDDYYDESKRIREGVETHVSFREHNLYTVNITFLEVKMEDYGLPFMCHA
GVSTAYIILQLPAPDFR
**SEQ ID NO:25** 
DGCNDIFMKNEMLSASQPFAFNCTFPPITSGEVSVTWYKNSSKIPVSKIIQSRIHQDETWI
LFLPMEWGDSGVYQCVIKGRDSCHRIHVNLTVFEKQWCDTSVKGLPNLSDEYKQILHL
GKDDSLTCHLHFPKSCILGPIKWYKDCNEITGERFTVLETRLLVSNVSAEDRGNYACQVI
LTHSGKQYEVLNGITVSITERAGYGGSVPKIIYPKNNSIEVQLGTTLIVDCNITDTKDNTN
LRCWRVNNTLVDDYYNESKRIREGVETHASFREYNLYTVNITFLEVKMEDYGLPFMCH
AGVSAAYIMLQLPAPDFR
**SEQ ID NO:26** 
SERCDDWGLDTMRQIQVFEDEPARIKCPLFEHFLKFNYSTAHSAGLTLIWYWTRQDRDL
EEPINFRLPENRISKEKDVLWFRPTLLNDTGNYTCMLRNTTYCSKVAFPLEVVQKDSCFN
SPMKLPVHKLYIEYGIQRITCPNVDGYFPSSVKPTITWYMGCYKIQNFNNVIPEGMNLSF
LIALISNNGNYTCVVTYPENGRTFHLTRTLTVKVVGSPKNAVPPVIHSPNDHVVYEKEPG
EELLIPCTVYFSFLMDSRNEVWWTIDGKKPDDITIDVTINESISHSRTEDETRTQILSIKKV
TSEDLKRSYVCHARSAKGEVAKAAKVKQKVPAPRYTVE
**SEQ ID NO:27** 
SERCDDWGLDTMRQIQVFEDEPARIKCPLFEHFLKFNYSTAHSAGLTLIWYWTRQDRDL
EEPINFRLPENRISKEKDVLWFRPTLLNDTGNYTCMLRNTTYCSKVAFPLEVVQKDSCFN
SPMKLPVHKLYIEYGIQRITCPNVDGYFPSSVKPTITWYMGCYKIQNFNNVIPEGMNLSF
LIAFISNNGNYTCVVTYPENGRTFHLTRTLTVKVVGSPKNAVPPVIHSPNDHVVYEKEPG
EELLIPCTVYFSFLMDSRNEVWWTIDGKKPDDIPIDVTINESISHSRTEDETRTQILSIKKV
TSEDLKRSYVCHARSAKGEVAKAATVKQKVPAPRYTVE
**SEQ ID NO:28** 
SAPFSFLSNVKYNFMRIIKYEFILNDALNQSIIRANDQYLTAAALHNLDEAVKFDMGAYK
SSKDDAKITVILRISKTQLYVTAQDEDQPVLLKEMPEIPKTITGSETNLLFFWETHGTKNY
FTSVAHPNLFIATKQDYWVCLAGGPPSITDFQILENQA
**SEQ ID NO:29** 
APVRSLNCTLRDSQQKSLVMSGPYELKALHLQGQDMEQQVVFSMSFVQGEESNDKIPV
ALGLKEKNLYLSCVLKDDKPTLQLESVDPKNYPKKKMEKRFVFNKIEINNKLEFESAQF
PNWYISTSQAENMPVFLGGTKGGQDITDFTMQFVSS
**SEQ ID NO:30** 
SITGISPITEYLASLSTYNDQSITFALEDESYEIYVEDLKKDEKKDKVLLSYYESQHPSNES
GDGVDGKMLMVTLSPTKDFWLHANNKEHSVELHKCEKPLPDQAFFVLHNMHSNCVSF
ECKTDPGVFIGVKDNHLALIKVDSSENLCTENILFKLSET
**SEQ ID NO:31** 
KIDTPQQGSIQDINHRVWVLQDQTLIAVPRKDRMSPVTIALISCRHVETLEKDRGNPIYL
GLNGLNLCLMCAKVGDQPTLQLKEKDIMDLYNQPEPVKSFLFYHSQSGRNSTFESVAFP
GWFIAVSSEGGCPLILTQELGKANTTDFGLTMLF
**SEQ ID NO:32** 
REAAPKSYAIRDSRQMVWVLSGNSLIAAPLSRSIKPVTLHLIACRDTEFSDKEKGNMVYL
GIKGKDLCLFCAEIQGKPTLQLKEKNIMDLYVEKKAQKPFLFFHNKEGSTSVFQSVSYPG
WFIATSTTSGQPIFLTKERGITNNTNFYLDSVE
**SEQ ID NO:33** 
SMCKPITGTINDLNQQVWTLQGQNLVAVPRSDSVTPVTVAVITCKYPEALEQGRGDPIY
LGIQNPEMCLYCEKVGEQPTLQLKEQKIMDLYGQPEPVKPFLFYRAKTGRTSTLESVAFP
DWFIASSKRDQPIILTSELGKSYNTAFELNIND
**SEQ ID NO:34**
   HHHHHH
**SEQ ID NO:35** 
PRGPTIKPCPPCKCPAPNLLGGPSVFIFPPKIKDVLMISLSPIVTCVVVDVSEDDPDVQISW
FVNNVEVHTAQTQTHREDYNSTLRVVSALPIQHQDWMSGKEFKCKVNNKDLPAPIERTI
SKPKGSVRAPQVYVLPPPEEEMTKKQVTLTCMVTDFMPEDIYVEWTNNGKTELNYKNT
EPVLDSDGSYFMYSKLRVEKKNWVERNSYSCSVVHEGLHNHHTTKSFSRTPGK
**SEQ ID NO:36** 
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPELLGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLYSKLTVDK
SRWQQGNVFSCSVMHEALHNHYTQKSLSLSPGK
**SEQ ID NO:37** ASTKGPSVFPLAPSSKSTSGGTAALGCLVKDYFPEPVTVSWNSGALTSGVHTFPAVLQSS 
GLYSLSSVVTVPSSSLGTQTYICNVNHKPSNTKVDKRVEPKSCDKTHTCPPCPAPEFEGGP
SVFLFPPKPKDTLMISRTPEVTCVVVDVSHEDPEVKFNWYVDGVEVHNAKTKPREEQY
NSTYRVVSVLTVLHQDWLNGKEYKCKVSNKALPASIEKTISKAKGQPREPQVYTLPPSR
EEMTKNQVSLTCLVKGFYPSDIAVEWESNGQPENNYKTTPPVLDSDGSFFLYSKLTVDK
SRWQQGNVFSCSVMHEALHNHYTQKSLSLSPGK
**SEQ ID NO:38** 
ASTKGPSVFPLAPSSKSTSEGTAALGCLVKDYFPEPVTVSWNSGALTSGVHTFPAVLQSS
GLYSLSSVVTVPSSSLGTQTYICNVDHKPSNTKVDKTVEPKSCDKTHTCPPCPAPEFEGG
PSVFLFPPKPKDTLMISRTPEVTCVVVDVSQEDPEVQFNWYVDGVEVHNAKTKPREEQY
NSTYRVVSVLTVLHQDWLNGKEYKCKVSNKALPASIEKTISKAKGQPREPQVCTLPPSQ
EEMTKNQVSLSCAVKGFYPSDIAVEWESNGQPENNYKTTPPVLDSDGSFFLVSKLTVDK
SRWQEGNVFSCSVMHEALHNHYTQKSLSLSPGK
**SEQ ID NO:39** 
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPEFEGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPASIEKTISKAKGQPREPQVYTLPPCR
EEMTKNQVSLWCLVKGFYPSDIAVEWESNGQPENNYKTTPPVLDSDGSFFLYSKLTVD
KSRWQQGNVFSCSVMHEALHNHYTQKSLSLSPGK
**SEQ ID NO:40**
**SEQ ID NO:41** 
GQPKAAPSVTLFPPSSEELQANKATLVCLISDFYPGAVTVAWKADSSPVKAGVETTTPS
KQSNNKYAASSYLSLTPEQWKSHRSYSCQVTHEGSTVEKTVAPTECS
**SEQ ID NO:42**
**SEQ ID NO:43**
**SEQ ID NO:44** 
QVQLVQSGAEVKKPGASVKVSCKASGYSFTSSWIHWVRQAPGQGLEWMGEINPGSGRS
NYNENFQGRVTMTVDTSISTAYMELSRLRSDDTAVYYCTVVFYGEPYFPFWGQGTLVT
 
VSSASTKGPSVFPLAPSSKSTSEGTAALGCLVKDYFPEPVTVSWNSGALTSGVHTFPAVLQ
SSGLYSLSSVVTVPSSSLGTQTYICNVDHKPSNTKVDKTVEPKSCDKTHTCPPCPAPEFEG
GPSVFLFPPKPKDTLMISRTPEVTCVVVDVSQEDPEVQFNWYVDGVEVHNAKTKPREEQ
YNSTYRVVSVLTVLHQDWLNGKEYKCKVSNKALPASIEKTISKAKGQPREPQVCTLPPS
QEEMTKNQVSLSCAVKGFYPSDIAVEWESNGQPENNYKTTPPVLDSDGSFFLVSKLTVD
KSRWQEGNVFSCSVMHEALHNHYTQKSLSLSPGK
**SEQ ID NO:45** 
EVQLVESGGGLVQPGGSLRLSCAASGFTFSSYAMSWVRQAPGKGLVWVAEISRGGSHT
YYPDTVTGRFTISRDNAKNTLYLQMNSLRAEDTAVYYCARSYGNYPFDYWGQGTTVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPEF
EGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPASIEKTISKAKGQPREPQVYTLP
PCREEMTKNQVSLWCLVKGFYPSDIAVEWESNGQPENNYKTTPPVLDSDGSFFLYSKLT
VDKSRWQQGNVFSCSVMHEALHNHYTQKSLSLSPGK
**SEQ ID NO:46** 
EIVLTQSPGTLSLSPGERATLSCRASQSVIGSYLAWYQQKPGQAPRLLIYSASKLASGIPD
RFSGSGSGTDFTLTISRLEPEDFAVYYCQQDTRYPITFGQGTKVEIKRTVAAPSVFIFPPSD
EQLKSGTASVVCLLNNFYPREAKVQWKVDNALQSGNSQESVTEQDSKDSTYSLSSTLTL
SKADYEKHKVYACEVTHQGLSSPVTKSFNRGEC
**SEQ ID NO:47** 
EIVLTQSPGTLSLSPGERATLSCRASQSVIYSYLAWYQQKPGQAPRLLIYSASKRASGIPD
RFSGSGSGTDFTLTISRLEPEDFAVYYCQQHAGIPVTFGQGTKVEIKRTVAAPSVFIFPPSD
EQLKSGTASVVCLLNNFYPREAKVQWKVDNALQSGNSQESVTEQDSKDSTYSLSSTLTL
SKADYEKHKVYACEVTHQGLSSPVTKSFNRGEC
**SEQ ID NO:48** 
EVQLVESGGGLVQPGGSLRLSCAASGFTFSSYAMSWVRQAPGKGLVWVAEISRGGSHT
YYPDTVTGRFTISRDNAKNTLYLQMNSLRAEDTAVYYCARSYGNYPFDYWGQGTTVT
VSSASVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGECDKTHTCPP
CPAPEFEGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPASIEKTISKAKGQPREP
QVYTLPPCREEMTKNQVSLWCLVKGFYPSDIAVEWESNGQPENNYKTTPPVLDSDGSFF
LYSKLTVDKSRWQQGNVFSCSVMHEALHNHYTQKSLSLSPGK
**SEQ ID NO:49** 
EIVLTQSPATLSLSPGERATLSCSASSSVSYMYWYQQKPGQAPRLLIYDTSNLASGIPARFS
GSGSGTDFTLTISSLEPEDFAVYYCQQWSSYPLTFGQGTKLEIKSSASTKGPSVFPLAPSSK
STSGGTAALGCLVKDYFPEPVTVSWNSGALTSGVHTFPAVLQSSGLYSLSSVVTVPSSSLG
TQTYICNVNHKPSNTKVDKRVEPKSC
**SEQ ID NO:50** 
QVQLVQSGAEVKKPGASVKVSCKASGYSFTSSWIHWVKQAPGQGLEWMGEINPGNVR
SNYNENFRNKVTMTVDTSISTAYMELSRLRSDDTAVYYCTVINYGEPYFASWGQGTLV
TVSSASTKGPSVFPLAPSSKSTSEGTAALGCLVKDYFPEPVTVSWNSGALTSGVHTFPAV
LQSSGLYSLSSVVTVPSSSLGTQTYICNVDHKPSNTKVDKTVEPKSCDKTHTCPPCPAPE
FEGGPSVFLFPPKPKDTLMISRTPEVTCVVVDVSQEDPEVQFNWYVDGVEVHNAKTKPR
EEQYNSTYRVVSVLTVLHQDWLNGKEYKCKVSNKALPASIEKTISKAKGQPREPQVCTL
PPSQEEMTKNQVSLSCAVKGFYPSDIAVEWESNGQPENNYKTTPPVLDSDGSFFLVSKL
TVDKSRWQEGNVFSCSVMHEALHNHYTQKSLSLSPGK
**SEQ ID NO:51** 
EVKLVESGGGLVKPGGSLKLSCAASGFTFSSYAMSWVRQSPEKRLEWVAEISRGGSHTY
YPDTVTGRFTISRDNAKNTLYLEMTSLRSEDTALYYCARSYGNYPFDYWGQGTTLTVSS
**SEQ ID NO:52** 
DIVMTQSPAIMSASPGEKVTMTCSASSSVSYMYWYQQKPGSSPRLLIYDTSNLASGVPV
RFSGSGSGTSYSLTISRMEAEDAATYYCQQWSSYPLTFGAGTKLEIK
**SEQ ID NO:53** 
EVQLLESGGGLVQPGGSLRLSCAASGFTFSSYAMSWVRQAPGKGLEWVSAISGSGGSTY
YADSVKGRFTISRDNSKNTLYLQMNSLRAEDTAVYYCAKGSGFDYWGQGTLVTVSS
**SEQ ID NO:54** 
EIVLTQSPGTLSLSPGERATLSCRASQSVSSSYLAWYQQKPGQAPRLLIYGASSRATGIPD
RFSGSGSGTDFTLTISRLEPEDFAVYYCQQYGSSPLTFGQGTKVEIK
**SEQ ID NO:55**
   TGCATTTGAACTCCTTGCC
**SEQ ID NO:56**
   CCATCAATCTTCCACTTGAC

## Claims

1. A bispecific antibody comprising a first antigen-binding fragment that binds to human IL-36R and a second antigen-binding fragment that binds to human IL-1R3;
optionally, the bispecific antibody is capable of inhibiting an IL-36-mediated inflammatory response, such as inhibiting binding of IL-36α, IL-36β and/or IL-36γ to IL-36R, inhibiting SEAP secretion by a cell, and/or inhibiting IL-8 secretion by a cell.

2. The bispecific antibody of claim 1, wherein
the first antigen-binding fragment comprises:
HCDR1 having the amino acid sequence SSWIH (SEQ ID NO: 1), HCDR2 having the amino acid sequence EINPGNVRSNYNENFRN (SEQ ID NO: 2), HCDR3 having the amino acid sequence INYGEPYFAS (SEQ ID NO: 3), LCDR1 having the amino acid sequence RASQSVIGSYLA (SEQ ID NO: 4), LCDR2 having the amino acid sequence SASKLAS (SEQ ID NO: 5), and LCDR3 having the amino acid sequence QQDTRYPIT (SEQ ID NO: 6); or
HCDR1 having the amino acid sequence SSWIH (SEQ ID NO: 1), HCDR2 having the amino acid sequence EINPGSGRSNYNENFQG (SEQ ID NO: 7), HCDR3 having the amino acid sequence VFYGEPYFPF (SEQ ID NO: 8), LCDR1 having the amino acid sequence RASQSVIYSYLA (SEQ ID NO: 9), LCDR2 having the amino acid sequence SASKRAS (SEQ ID NO: 10), and LCDR3 having the amino acid sequence QQHAGIPVT (SEQ ID NO: 11); and/or
the second antigen-binding fragment comprises:
HCDR1 having the amino acid sequence SYAMS (SEQ ID NO: 12), HCDR2 having the amino acid sequence EISRGGSHTYYPDTVTG (SEQ ID NO: 13), HCDR3 having the amino acid sequence SYGNYPFDY (SEQ ID NO: 14), LCDR1 having the amino acid sequence RASQSVIGSYLA (SEQ ID NO: 4), LCDR2 having the amino acid sequence SASKLAS (SEQ ID NO: 5), and LCDR3 having the amino acid sequence QQDTRYPIT (SEQ ID NO: 6); or
HCDR1 having the amino acid sequence SYAMS (SEQ ID NO: 12), HCDR2 having the amino acid sequence EISRGGSHTYYPDTVTG (SEQ ID NO: 13), HCDR3 having the amino acid sequence SYGNYPFDY (SEQ ID NO: 14), LCDR1 having the amino acid sequence SASSSVSYMY (SEQ ID NO: 15), LCDR2 having the amino acid sequence DTSNLAS (SEQ ID NO: 16), and LCDR3 having the amino acid sequence QQWSSYPLT (SEQ ID NO: 17);
wherein the amino acid sequences of the HCDRs and the LCDRs are defined according to Kabat.

3. The bispecific antibody of claim 2, wherein
the first antigen-binding fragment comprises a heavy chain variable region having the amino acid sequence set forth in SEQ ID NO: 18, and a light chain variable region having the amino acid sequence set forth in SEQ ID NO: 19; or
the first antigen-binding fragment comprises a heavy chain variable region having the amino acid sequence set forth in SEQ ID NO: 20, and a light chain variable region having the amino acid sequence set forth in SEQ ID NO: 21; and/or
the second antigen-binding fragment comprises a heavy chain variable region having the amino acid sequence set forth in SEQ ID NO: 22, and a light chain variable region having the amino acid sequence set forth in SEQ ID NO: 19; or
the second antigen-binding fragment comprises a heavy chain variable region having the amino acid sequence set forth in SEQ ID NO: 22, and a light chain variable region having the amino acid sequence set forth in SEQ ID NO: 23.

4. The bispecific antibody of any one of claims 1-3, wherein
the first antigen-binding fragment comprises a heavy chain variable region set forth in SEQ ID NO: 18 and a light chain variable region set forth in SEQ ID NO: 19, and the second antigen-binding fragment comprises a heavy chain variable region set forth in SEQ ID NO: 22 and a light chain variable region set forth in SEQ ID NO: 19; or
the first antigen-binding fragment comprises a heavy chain variable region set forth in SEQ ID NO: 20 and a light chain variable region set forth in SEQ ID NO: 21, and the second antigen-binding fragment comprises a heavy chain variable region set forth in SEQ ID NO: 22 and a light chain variable region set forth in SEQ ID NO: 23;
preferably, the first antigen-binding fragment comprises a heavy chain set forth in SEQ ID NO: 50 and a light chain set forth in SEQ ID NO: 46, and the second antigen-binding fragment comprises a heavy chain set forth in SEQ ID NO: 45 and a light chain set forth in SEQ ID NO: 46; or
the first antigen-binding fragment comprises a heavy chain set forth in SEQ ID NO: 44 and a light chain set forth in SEQ ID NO: 47, and the second antigen-binding fragment comprises a heavy chain set forth in SEQ ID NO: 48 and a light chain set forth in SEQ ID NO: 49.

5. The bispecific antibody of any one of claims 1-4, wherein
the bispecific antibody is an IgG1 antibody comprising a first heavy chain constant region and a second heavy chain constant region, wherein
amino acids at positions 354 and 366 of the first heavy chain constant region are C and W, respectively, and amino acids at positions 349, 366, 368 and 407 of the second heavy chain constant region are C, S, A and V, respectively; and/or
amino acids at positions 234, 235 and 331 of the first heavy chain constant region and the second heavy chain constant region are F, E and S, respectively;
the amino acid positions of the antibody constant regions are determined according to EU numbering.

6. The bispecific antibody of any one of claims 1-5, wherein
the first antigen-binding fragment and the second antigen-binding fragment are independently selected from a single chain fragment variable (scFv) or a Fab fragment; preferably, both of the first antigen-binding fragment and the second antigen-binding fragment are Fab fragments.

7. A monoclonal antibody that binds to human IL-36R, comprising
HCDR1 set forth in SEQ ID NO: 1,
HCDR2 set forth in SEQ ID NO: 7,
HCDR3 set forth in SEQ ID NO: 8,
LCDR1 set forth in SEQ ID NO: 9,
LCDR2 set forth in SEQ ID NO: 10, and
LCDR3 set forth in SEQ ID NO: 11; or
HCDR1 set forth in SEQ ID NO: 1,
HCDR2 set forth in SEQ ID NO: 2,
HCDR3 set forth in SEQ ID NO: 3,
LCDR1 set forth in SEQ ID NO: 4,
LCDR2 set forth in SEQ ID NO: 5, and
LCDR3 set forth in SEQ ID NO: 6; or
HCDR1 set forth in SEQ ID NO: 1,
HCDR2 set forth in SEQ ID NO: 7,
HCDR3 set forth in SEQ ID NO: 8,
LCDR1 set forth in SEQ ID NO: 4,
LCDR2 set forth in SEQ ID NO: 5, and
LCDR3 set forth in SEQ ID NO: 6;
wherein the amino acid sequences of the HCDRs and the LCDRs are defined according to Kabat;
preferably, the antibody has a heavy chain variable region having the amino acid sequence set forth in SEQ ID NO: 20, and a light chain variable region having the amino acid sequence set forth in SEQ ID NO: 21; or
the antibody has a heavy chain variable region having the amino acid sequence set forth in SEQ ID NO: 18, and a light chain variable region having the amino acid sequence set forth in SEQ ID NO: 19; or
the antibody has a heavy chain variable region having the amino acid sequence set forth in SEQ ID NO: 20, and a light chain variable region having the amino acid sequence set forth in SEQ ID NO: 19.

8. A monoclonal antibody that binds to human IL-1R3, comprising
HCDR1 set forth in SEQ ID NO: 12,
HCDR2 set forth in SEQ ID NO: 13,
HCDR3 set forth in SEQ ID NO: 14,
LCDR1 set forth in SEQ ID NO: 15,
LCDR2 set forth in SEQ ID NO: 16, and
LCDR3 set forth in SEQ ID NO: 17;
wherein the amino acid sequences of the HCDRs and the LCDRs are defined according to Kabat;
preferably, the antibody has a heavy chain variable region having the amino acid sequence set forth in SEQ ID NO: 22 and a light chain variable region having the amino acid sequence set forth in SEQ ID NO: 23; or
the antibody has a heavy chain variable region having the amino acid sequence set forth in SEQ ID NO: 51, and a light chain variable region having the amino acid sequence set forth in SEQ ID NO: 52.

9. A pharmaceutical composition comprising the bispecific antibody of any one of claims 1-6, or the monoclonal antibody of claim 7 or 8, and a pharmaceutically acceptable excipient, diluent or carrier.

10. A pharmaceutical composition of claim 9, for use in the prevention or treatment of an IL-36-mediated disease; preferably, the IL-36-mediated disease is selected from the group consisting of psoriasis vulgaris, inflammatory bowel disease, renal injury and inflammation, pneumonia, arthritis, hidradenitis suppurative, generalized pustular psoriasis, and palmoplantar pustular psoriasis.

11. Use of the bispecific antibody of any one of claims 1-6, the monoclonal antibody of claim 7 or 8, or the pharmaceutical composition of claim 9 or 10 in the manufacture of a medicament for the prevention or treatment of an IL-36-mediated disease; preferably, the IL-36-mediated disease is selected from the group consisting of psoriasis vulgaris, inflammatory bowel disease, renal injury and inflammation, pneumonia, arthritis, hidradenitis suppurative, generalized pustular psoriasis, and palmoplantar pustular psoriasis.

12. A method of preventing or treating an IL-36-mediated disease comprising administering to a subject in need thereof the bispecific antibody of any one of claims 1-6, the monoclonal antibody of claim 7 or 8, or the pharmaceutical composition of claim 9 or 10; preferably, the IL-36-mediated disease is selected from the group consisting of psoriasis vulgaris, inflammatory bowel disease, renal injury and inflammation, pneumonia, arthritis, hidradenitis suppurative, generalized pustular psoriasis, and palmoplantar pustular psoriasis.
